# EUROPEAN PATENT SPECIFICATION

(11) **EP 4 385 460 B1**
(45) Date of publication and mention of the grant of the patent: **04.11.2026**
(21) Application number: 22874906.5
(22) Date of filing: 27.09.2022
(51) Int. Cl.: A61F 2/24

(54) **PROSTHETIC VALVE REPLACEMENT SYSTEM**
PROTHESENKLAPPENERSATZSYSTEM
SYSTÈME DE REMPLACEMENT DE VALVE PROTHÉTIQUE

(30) Priority: 30.09.2021 CN 202111164164
(43) Date of publication of application: 19.06.2024
(73) Proprietor: Jenscare Scientific Co., Ltd, Ningbo, Zhejiang 315336 (CN)
(72) Inventor: LV, Shiwen, Hangzhou Bay New District Ningbo, Zhejiang 315336 (CN); CHEN, Zhi, Hangzhou Bay New District Ningbo, Zhejiang 315336 (CN); CHEN, Jinxiong, Hangzhou Bay New District Ningbo, Zhejiang 315336 (CN); ZHANG, Shandong, Hangzhou Bay New District Ningbo, Zhejiang 315336 (CN)
(74) Representative: Weickmann & Weickmann PartmbB
(86) International application number: PCT/CN2022/121698
(87) International publication number: WO 2023/051521

(56) References cited:
- CN-A- 103 237 523
- CN-A- 111 110 398
- CN-A- 111 568 607
- CN-A- 112 704 580
- CN-A- 113 855 329
- US-A1- 2013 035 759
- US-A1- 2014 207 231
- US-A1- 2016 184 095
- US-A1- 2016 346 080

## Description

### CROSS-REFERENCE TO RELATED APPLICATIONS

This application claims priority to patent application No. 202111164164.4 filed on Sep. 30, 2021.

### TECHNICAL FIELD

The present application relates to the field of medical devices and, in particular, to a prosthetic valve replacement system.

### BACKGROUND

From the perspective of the structure of a heart, both a mitral valve and a tricuspid valve have a special physiological structure, making accurate positioning and securing of a product very difficult. In particular, the specific anatomical position and complex anatomical structure of the mitral valve within the heart pose great challenges to a mitral valve replacement surgery.

In the related art, a technique for surgically locking a mitral valve/tricuspid valve prosthesis within the heart often utilizes a radial support force of a stent to an atrioventricular valve annulus. Disadvantages of this technique are that tissue surrounding the valve annulus is prone to be compressed and that the stent and the compressed tissue are prone to affect an outflow tract. From the perspective of a long-term use effect of the replacement valve, as the use time of a patient goes by after surgery, regurgitation decreases, ventricular atrial pressure decreases, the structure of the heart is reconstructed, and a relatively large-size stent affects the reduction in the size of the valve annulus, thereby affecting the internal structure of the heart. Apparently, this is not a desirable result.

In addition, the locking technique may also be that a docking apparatus is pre-implanted in a valve leaflet and then the stent is opened inside the docking apparatus and thus is secured at the valve leaflet. Patent CN109789019A describes a heart valve docking coil and system from Edwards Lifesciences. In the disclosure of the patent, the valve leaflet and the stent are tightly hooped by winding the docking apparatus around the external side of the valve leaflet. This manner of stent securing is outstandingly advantageous, but the blood flow blockage of a left outflow tract is not addressed due to a relatively large anterior valve leaflet hooped on the stent. The documents US 2013/035759 A1, US 2014/207231 and US 2016/346080 A1 also disclose heart valve replacement systems in that the autologous valve leaflets are held at the outside of the valve stent by clips and/or a hoop.

**In** conclusion, a mitral valve replacement prosthesis currently has at least the following technical pain points: 1. After a prosthesis replacement, the anterior valve leaflet blocks the left ventricular outflow tract; 2. the radial support of an autologous annulus by a large-size stent affects the internal structure of the heart, resulting in a large number of complications. A new transcatheter heart valve replacement system is urgently needed in the industry to address the preceding and other technical problems.

### SUMMARY

According to embodiments of the present application, the present application provides a prosthetic valve replacement system. A main object of the present application is to overcome some problems and shortcomings in the related art.

According to an aspect of the present application, a prosthetic valve replacement system is provided. The prosthetic valve replacement system includes a valve stent and a valve clamping and securing apparatus cooperating with the valve stent, where the valve clamping and securing apparatus is constructed as a valve clamping mechanism and a hoop member for clamping autologous valve leaflets, and the hoop member is disposed on the valve clamping mechanism, where the valve clamping and securing apparatus is configured to be able to present a first form and a second form, and the second form is present after the first form, where when the valve clamping and securing apparatus is in the first form, the valve clamping mechanism is configured to be able to capture and clamp the autologous valve leaflets, and where after a radial expansion of the valve stent, the valve clamping and securing apparatus is in the second form, and the radial expansion of the valve stent causes the hoop member to drive the autologous valve leaflets to move upward and tightly hoop the valve stent.

According to an embodiment, a far end of the valve clamping mechanism is detachably connected, and under the first form, the valve clamping and securing apparatus allows a valve leaflet around the valve clamping mechanism to be able to open and close autonomously, when the valve stent enters the valve clamping mechanism, the far end of the valve clamping mechanism is detached, and when the valve stent is in a radial expansion state, the radial expansion of the valve stent causes the hoop member to drive the autologous valve leaflets to move upward and tightly hoop the valve stent.

According to an embodiment, after the radial expansion of the valve stent, the radial expansion of the valve stent drives the valve clamping mechanism, the hoop member, the autologous valve leaflets and chordae tendineae in linkage, where the hoop member is at least partially moved upward and drives the autologous valve leaflets and the chordae tendineae to be lifted upward, and the hoop member tightly hoops the valve stent, and the valve clamping and securing apparatus is in the second form at this time.

According to an embodiment, under the first form, the valve clamping mechanism clamps a portion of each of the autologous valve leaflets, and under the second form, the autologous valve leaflets around the valve clamping mechanism are driven by the hoop member to move upward.

According to an embodiment, the hoop member is a closed-loop structure and passes through the valve clamping mechanism.

According to another embodiment, the hoop member is connected to the valve clamping mechanism and forms a closed-loop structure, and the hoop member is two segments of threads or silks, and two ends of the hoop member are securely connected to the valve clamping mechanism.

According to an embodiment, the hoop member and the valve clamping mechanism include at least two connecting points, and the at least two connecting points of the hoop member and the valve clamping mechanism have different heights.

According to another embodiment, when the prosthetic valve replacement system is configured to replace a mitral valve, the at least two connecting points of the hoop member on the valve clamping mechanism have different heights. Specifically, the height of the hoop member in an anterior valve leaflet region is greater than the height of the hoop member in a posterior valve leaflet region. This can not only lift an anterior valve leaflet outstandingly and avoid blocking a left ventricular outflow tract but also prevent the hoop member from excessively pulling chordae tendineae in the posterior valve leaflet region to protect chordae tendineae tissue.

According to another embodiment, the valve clamping mechanism includes a plurality of clamps, and connecting points of the hoop member and the plurality of clamps have different heights to enable the hoop member to have a waved form under the second form or to enable the height of the hoop member in the anterior valve leaflet region to be greater than the height of the hoop member in the posterior valve leaflet region under the second form. This can lift the anterior valve leaflet to a relatively high position and prevent the anterior valve leaflet from blocking the left ventricular outflow tract after the replacement.

According to an embodiment, positions of the at least two connecting points of the hoop member and the valve clamping mechanism cannot be too high. For example, the positions of the at least two connecting points in the region of two thirds of the close end of each of the autologous valve leaflets can effectively prevent the hoop member from excessively pulling the chordae tendineae tissue.

According to an embodiment, when the valve clamping and securing apparatus is in the second form, the hoop member is located between the chordae tendineae and the autologous valve leaflets, and the radial expansion of the valve stent causes the hoop member to be at least partially moved upward and drive the autologous valve leaflets and the chordae tendineae to be lifted upward.

According to an embodiment, when the valve clamping and securing apparatus is in the second form, the hoop member is fastened to the outer periphery of the valve clamping mechanism in a tensioning state, and after being lifted upward by the hoop member, the autologous valve leaflets are folded back to at least partially overlap themself.

According to an embodiment, under the second form, the hoop member further tightly hoops the valve clamping mechanism and the autologous valve leaflets around the outer periphery of the valve stent circumferentially.

According to an embodiment, in the process of transitioning the valve clamping and securing apparatus from the first form to the second form, the valve stent is radially expanded gradually from a non-radial expansion state and gradually opens the valve clamping and securing apparatus so that the hoop member can be gradually unfolded and tensioned from a relaxing state, and thereby the chordae tendineae and the clamped autologous valve leaflets can be gradually lifted, and the valve stent and the valve clamping mechanism can be hooped tightly.

According to an embodiment, in the process of capturing and clamping the autologous valve leaflets by the valve clamping mechanism, the valve clamping mechanism moves toward the direction of an atrium so that the portions of the autologous valve leaflets and the chordae tendineae can be folded back inside a valve leaflet receiving cavity formed by the valve clamping mechanism and/or between the lower part of an autologous valve annulus and the hoop member.

According to an embodiment, under the first form, one end of the valve clamping mechanism abuts onto the roots of the clamped autologous valve leaflets.

According to an embodiment, the valve clamping and securing apparatus includes a single valve clamp, an atrial support segment (after the valve clamping and securing apparatus is mounted in position, the atrial support segment is located inside the atrium and supplies a support force), a ventricular segment and the hoop member, where the hoop member is connected to the single valve clamp and the ventricular segment, and the hoop member has an inclined form on a cross section parallel to a central axis of the valve clamping and securing apparatus. More specifically, the height of a joint between the hoop member and the single valve clamp is higher than the height of a portion of the hoop member region. When applied in a mitral valve treatment, the single valve clamp is configured to clamp the anterior valve leaflet, and the hoop member can outstandingly pull the anterior valve leaflet and related chordae tendineae tissue.

According to an embodiment, the valve clamping mechanism includes a first clamping half part and a second clamping half part, the first clamping half part and the second clamping half part are detachably connected or are two separate members separated from each other, and the first clamping half part and the second clamping half part are configured to capture and clamp different autologous valve leaflets respectively, and the perimeter of the closed loop formed by the hoop member is slightly less than the perimeter of the valve stent that is radially expanded in position.

According to an embodiment, the first clamping half part and the second clamping half part form a substantially sleeve-shaped configuration under the restraint state of the valve clamping and securing apparatus, and the substantially sleeve-shaped configuration is sleeved on an inner core tube and is sleeved on the outer periphery by an outer sheath tube.

According to an embodiment, the first clamping half part and the second clamping half part have complementary shapes and are detachably buckled under the restraint state of the valve clamping and securing apparatus.

According to an embodiment, the first clamping half part has a first support segment, a first detachable cooperation segment extending from the first support segment and a first clamping segment further extending from the first detachable cooperation segment, and the first support segment is provided with a first clamping jaw; the second clamping half part has a second support segment, a second detachable cooperation segment extending from the second support segment and a second clamping segment further extending downward from the first detachable cooperation segment, and the second support segment is provided with a second clamping jaw.

According to an embodiment, the first clamping half part and the second clamping half part each include a valve leaflet capturing member and a valve pressing member, and the hoop member is connected to one end of the valve leaflet capturing member.

According to an embodiment, the first clamping jaw and the second clamping jaw are each provided with a tooth, teeth may limit relative positions between the first clamping half part, the second clamping half part and the different autologous valve leaflets, and the teeth are configured to grip and secure the clamped different autologous valve leaflets respectively.

According to an embodiment, the teeth may be barbs or other structures that may be locked with the different valve leaflets so that the first clamping half part and the second clamping half part can tightly grip the different autologous valve leaflets respectively.

According to another embodiment, the teeth may be disposed at one end of the first clamping half part and one end of the second clamping half part respectively, the teeth can "secure" the different autologous valve leaflets respectively to avoid a slipping case after the first clamping half part and the second clamping half part clamp the different autologous valve leaflets respectively.

According to an embodiment, the first clamping segment is connected to the first detachable cooperation segment with each other through a spring segment, the first clamping jaw is connected to the first support segment with each other through the spring segment; the second clamping segment is connected to the second detachable cooperation segment with each other through a spring segment, and the second clamping jaw is connected to the second support segment with each other through the spring segment.

According to an embodiment, the first clamping half part and the second clamping half part are each integrally formed.

According to an embodiment, the first clamping half part and the second clamping half part are each composed of a shape memory alloy.

According to an embodiment, the first clamping half part and the second clamping half part are each configured to form a valve leaflet receiving cavity, and when the first clamping half part and the second clamping half part capture and clamp the different autologous valve leaflets respectively, portions of the different autologous valve leaflets are folded back inside valve leaflet receiving cavities respectively.

According to an embodiment, when the first clamping half part and the second clamping half part are opened and capture the different autologous valve leaflets, the different autologous valve leaflets are located between the opening angle of the first clamping half part and the opening angle of the second clamping half part, and a delivery system is operable to move the entire valve clamping and securing apparatus upward toward the direction of the atrium at this time so that the portions of the different autologous valve leaflets can be folded between the first clamping half part and the second clamping half part. Specifically, tip portions of the different autologous valve leaflets are folded inside the valve leaflet receiving cavities respectively. This can effectively shorten the lengths of the different autologous valve leaflets and prevent the different autologous valve leaflets from blocking the left ventricular outflow tract.

According to an embodiment, a positioning ring positioned within the atrium may be further disposed on the valve stent, is configured to have a form that adapts to a physiological structure of an autologous valve annulus and is provided with an auxiliary securing rod.

According to an embodiment, three or four auxiliary securing rods are provided. This can not only supply a powerful support but also avoid excessively tensioning the original valve annulus and reduce implants.

According to an embodiment, the positioning ring is provided with a flexible sealing membrane, and after the valve stent is mounted in position, the sealing membrane fits atrium tissue.

According to an embodiment, a barb for auxiliary securing is disposed on the valve stent and on the positioning ring.

According to an embodiment, a securing clamp is disposed on the valve stent, and under the second form, the securing clamp clamps the autologous valve leaflets and enables the autologous valve leaflets to be clamped between the valve stent and the securing clamp.

According to an embodiment, the prosthetic valve replacement system further includes the inner core tube and the outer sheath tube; the valve clamping and securing apparatus is configured to further present a restraint state when being restrained inside the outer sheath tube and between the inner core tube and the outer sheath tube; and under the restraint state, the valve clamping and securing apparatus cannot clamp the autologous valve leaflets.

According to an embodiment, the inner core tube is further included; and the valve stent may enter along the inner core tube and reach the middle region of the valve clamping mechanism.

According to an embodiment, the first clamping half part and the second clamping half part are detachably connected, the inner core tube is provided with an enlarged end, the first clamping half part is provided with the first detachable cooperation segment, and the second clamping half part is provided with the second detachable cooperation segment; and the first detachable cooperation segment and the second detachable cooperation segment are detachably connected through the inner core tube; and the enlarged end has a substantially cylindrical configuration, the first detachable cooperation segment and the second detachable cooperation segment may be embedded to each other to form a buckle-like connection, and the enlarged end is inserted between the first detachable cooperation segment and the second detachable cooperation segment to form the detachable connection; when the enlarged end is withdrawn from between the first detachable cooperation segment and the second detachable cooperation segment, the valve stent supplies a radial expansion force to the first clamping half part and the second clamping half part so that the first clamping half part and the second clamping half part can be detached and separated.

According to an embodiment, when the valve stent is in the middle region of the valve clamping mechanism, the enlarged end is withdrawn toward the close end with respect to the valve clamping mechanism so that the enlarged end can be detached and separated from the valve clamping mechanism.

According to an embodiment, the valve stent may be a balloon-expandable stent or a self-expandable stent.

According to an embodiment, the hoop member is substantially in the shape of a bar, a filament or a twine.

According to an embodiment, the outer periphery of the hoop member is covered with a membrane that can increase a friction force with the autologous valve leaflets.

According to another aspect of the present application, a prosthetic valve replacement system is further provided. The prosthetic valve replacement system includes a valve clamping and securing apparatus; a valve clamping mechanism that has a substantially sleeve-shaped configuration formed by a first clamping half part and a second clamping half part in a restraint state; a hoop member that is fastened to a far end of the valve clamping mechanism and forms a closed loop surrounding the valve clamping mechanism; an inner core tube; an outer sheath tube; and a tubular valve stent that is radially expandable, where under a restraint state of the valve clamping and securing apparatus, the valve clamping mechanism is sleeved on the inner core tube and is restrained inside the outer sheath tube; where under a first form of the valve clamping and securing apparatus, the first clamping half part and the second clamping half part are exposed from the outer sheath tube and are deformed to configurations that each can clamp an autologous valve leaflet, and the hoop member is in a relaxing state; and where under a second form of the valve clamping and securing apparatus, the valve stent is in a radial expansion state and is positioned between the first clamping half part and the second clamping half part, and the hoop member is tensioned by the radially expanded valve stent and thereby tightly hoops the valve stent and the first clamping half part and the second clamping half part that are fastened to the valve stent.

According to an embodiment, under the first form, the autologous valve leaflets maintain the function of locally opening and closing, and under the second form, at least a portion of each of the autologous valve leaflets is folded.

According to an embodiment, the prosthetic valve replacement system further provides a positioning ring, and the positioning ring is configured to have a form that adapts to a physiological structure of an autologous valve annulus.

According to an embodiment, the positioning ring and the valve clamping and securing apparatus are integral.

According to another aspect of the present application, a method for operating a prosthetic valve replacement system is further provided. The method includes the following steps. An outer sheath tube of the prosthetic valve replacement system is operated to enter a surgical site. The outer sheath tube is gradually withdrawn toward a close end with respect to an inner core tube and gradually exposes a valve clamping mechanism in the outer sheath tube in a restraint state, and as the outer sheath tube is withdrawn, a first clamping half part and a second clamping half part of the valve clamping mechanism that are composed of a shape memory alloy are gradually released and are gradually deformed to capture and clamp autologous valve leaflets respectively. The outer sheath tube is completely withdrawn from the surgical site, and the inner core tube is left in the original position. A valve stent delivery apparatus is operated to move a tubular valve stent along the inner core tube into and reach between the first clamping half part and the second clamping half part of the valve clamping mechanism, and the inner core tube is withdrawn. The valve stent is radially expanded, thereby the valve clamping mechanism is radially expanded, so a hoop member that is fastened to the outer periphery of the valve clamping mechanism is unfolded and tensioned. The unfolding and tensioning of the hoop member gradually lift chordae tendineae and autologous valve leaflets along the axial direction of the valve stent until the hoop member tightly hoops the valve stent and the valve clamping mechanism, and the autologous valve leaflets are finally lifted and folded back. The valve stent delivery apparatus is withdrawn.

According to an aspect of the present application, a prosthetic valve replacement system is provided. The prosthetic valve replacement system includes a valve stent and a valve clamping and securing apparatus, the valve clamping and securing apparatus includes a valve clamping mechanism and a hoop member, and the hoop member is disposed on the valve clamping mechanism, where the valve clamping and securing apparatus has a first form and a second form; when the valve clamping and securing apparatus is in the first form, the valve clamping mechanism is configured to be pre-secured to the middle region of an autologous valve in a manner of locally clamping autologous valve leaflets, the hoop member is in a relaxing form, and the autologous valve leaflets can maintain the function of locally opening and closing; and when the valve clamping and securing apparatus is in the second form, the valve stent is radially expanded and drives the valve clamping mechanism and the hoop member to be unfolded synchronously, the hoop member gradually lifts chordae tendineae, the autologous valve leaflets and adjacent tissue, and the autologous valve leaflets are finally folded back.

According to an embodiment, when the valve stent is radially expanded and drives the valve clamping mechanism and the hoop member to be unfolded synchronously, the hoop member pulls the autologous valve leaflets and the chordae tendineae to move toward the directions of connecting points of the hoop member and the valve clamping mechanism and drives a portion of the autologous valve leaflets to be folded back; and finally, the hoop member secures the portion of the autologous valve leaflets, the chordae tendineae and the valve clamping mechanism around the outer periphery of the valve stent.

According to an embodiment, in the process of capturing and clamping the autologous valve leaflets by the valve clamping mechanism, the valve clamping mechanism moves toward the direction of an atrium so that the portion of the autologous valve leaflets and the chordae tendineae can be folded back inside the valve clamping mechanism and/or between the lower part of an autologous valve annulus and the hoop member. This can lift the height of the valve leaflet and avoid blocking the left ventricular outflow tract.

According to an embodiment, a first clamping half part and a second clamping half part are detachably connected and may capture and clamp different autologous valve leaflets respectively.

According to another embodiment, the first clamping half part and the second clamping half part are two separate members and are arranged axially within an outer sheath tube, and the first clamping half part and the second clamping half part can independently capture and clamp the different autologous valve leaflets respectively.

Compared with the related art, advantages and beneficial technical effects of the present application include at least the following list.
1. After implantation surgery, a traditional valve prosthesis is mostly anchored by relying on the radial support force applied to the autologous valve annulus. This compresses the original valve annulus and is not conducive for long-term use. Defects in this anchoring are noticeable. Moreover, an existing solution of anchoring the stent by use of the valve leaflet cannot solve the problem of blocking the left ventricular outflow tract. According to an embodiment of the present application, the valve clamping mechanism is configured to capture and clamp the autologous valve leaflets to achieve an "edge-to-edge" repair. This can effectively reduce the regurgitation of the valve, and before the replaced valve stent is implanted, the autologous valve can still function normally so that more time can be obtained for the surgery, and complications can be effectively reduced. Meanwhile, the valve stent can smoothly reach the middle region of the valve clamping mechanism along the inner core tube, and this is accurate in positioning and more convenient to operate. When the valve stent is radially expanded and drives the valve clamping mechanism and the hoop member to be unfolded synchronously, the hoop member pulls the chordae tendineae and the autologous valve leaflets to move toward the directions of the at least two connecting points of the hoop member and the valve clamping mechanism and drives the autologous valve leaflets to be lifted upward, preventing the autologous valve leaflets from blocking the left ventricular outflow tract after the replacement. Finally, the hoop member secures the portion of the autologous valve leaflets, the chordae tendineae and the valve clamping mechanism around the outer periphery of the valve stent in such a manner that the autologous valve annulus can be prevented from being radially supported, the form of the original valve can be prevented from being adversely affected, and two technical pain points related to the treatment of mitral valve replacement in the related art can be solved simultaneously, thereby enjoying a great clinical significance.
2. In the related art, the hoop member only plays a role in securing the valve stent but cannot solve the problem of blocking the left ventricular outflow tract by the autologous valve leaflets. Different from the related art, in an embodiment of the present application, the valve clamping mechanism is first anchored on the autologous valve leaflets, the at least two connecting points of the hoop member and the valve clamping mechanism are higher than the joint of the tip portion of the autologous valve leaflets and the chordae tendineae tissue, and the hoop member has a fixed length. When the valve stent is radially expanded, the hoop member moves upward toward the positions of the at least two connecting points of the hoop member and the valve clamping mechanism. In this process, the hoop member uses the at least two connecting points as fulcrums and pulls the chordae tendineae to further drive the autologous valve leaflets to be lifted upward so that the hoop member can tightly hoop the valve stent finally, thereby preventing the hoop member from radially supporting the autologous valve annulus, effectively preventing the autologous valve leaflets from blocking the outflow tract and enjoying a great clinical significance.
3. Different from the related art, in an embodiment of the present application, the end of the first clamping half part and the end of the second clamping half part are each provided with the valve leaflet receiving cavity so that the portion of the autologous valve leaflets can be folded back inside the valve leaflet receiving cavity when the valve clamping mechanism captures and clamps the valve leaflet, and the folded-back valve leaflet can have a shortened length. This effectively avoids blocking the left ventricular outflow tract and reduces the impact of original valve leaflet tissue on blood flow after the replacement. In particular, when applied in the mitral valve replacement, this is very suitable for the long physiological feature of the anterior valve leaflet of the mitral valve and enjoys a great clinical significance.
4. Different from the related art, in an embodiment of the present application, the positioning ring supplies a locking force to the valve clamping mechanism at the valve annulus or the atrium so that the valve clamping mechanism can be well maintained at a position within the heart when the first clamping half part and the second clamping half part capture and clamp the different valve leaflets respectively, and the valve stent has not been brought into the release position, preventing the valve clamping mechanism from slipping.
5. Different from the related art, in an embodiment of the present application, the inner core tube can not only control the detachable connection of the valve clamping mechanism but also serve as a guidance path for the valve stent to enter the valve clamping mechanism so that the operation time can be greatly shortened, and the operation accuracy can be improved. After the enlarged end only needs to be withdrawn from between the first detachable cooperation segment and the second detachable cooperation segment, the valve stent supplies the radial expansion force to the first clamping half part and the second clamping half part in the process of gradually radially expanding to restore the functional form so that the first clamping half part and the second clamping half part can be detached and separated. The structure is simple in assembly and convenient to disassemble, is conducive to the development of clinical surgery and enjoys a great clinical significance.
6. Different from the related art, in an embodiment of the present application, the at least two connecting points of the hoop member on the valve clamping mechanism have different heights. Specifically, the height of the hoop member in the anterior valve leaflet region is greater than the height of the hoop member in the posterior valve leaflet region. This can not only lift the anterior valve leaflet outstandingly and avoid blocking the left ventricular outflow tract but also prevent the hoop member from excessively pulling the chordae tendineae in the posterior valve leaflet region to protect the chordae tendineae tissue.

### BRIEF DESCRIPTION OF DRAWINGS

FIGS. 1A to 1C are views illustrating the structure of a valve stent, a valve clamping and securing apparatus and a hoop member according to an embodiment of the present application.
FIGS. 2A to 2J are views illustrating details and the operation principle of a valve clamping and securing apparatus according to an embodiment of the present application.
FIGS. 3A to 3D illustrate a hoop member according to another embodiment of the present application.
FIGS. 4A to 4D are views illustrating the process in which an outer sheath tube enters the heart according to an embodiment of the present application.
FIGS. 5A to 5K are views illustrating the process in which a valve clamping and securing apparatus and a hoop member cooperate with a valve stent to complete a valve replacement according to an embodiment of the present application.
FIGS. 6A to 6C illustrate one implementation of disposing a positioning ring according to an embodiment of the present application.
FIGS. 7A to 7H are views illustrating the operation principle of a positioning ring according to an embodiment of the present application.
FIG. 8A illustrates a positioning ring according to another embodiment of the present application.
FIGS. 9A to 9D are views illustrating a balloon-expandable stent according to another embodiment of the present application.
FIGS. 10A and 10B are views according to another embodiment of the present application.
FIGS. 11A and 11B are views according to another embodiment of the present application.
FIGS. 12A to 12C are views according to another embodiment of the present application.
FIGS. 13A and 13B are views according to another embodiment of the present application.

### Reference list

- 1: valve stent
- 11: positioning ring
- 2: valve clamping and securing apparatus
- 21: valve clamping mechanism
- 211: first clamping half part
- 2111: first detachable cooperation segment
- 2112: first support segment
- 212: second clamping half part
- 2121: second detachable cooperation segment
- 2122: second support segment
- 213: tooth
- 214: valve leaflet receiving cavity
- 215: expandable passage
- 22: capturing controller
- 23: detachable controller
- 24: positioning ring controller
- 25: clamp
- 3: hoop member
- 4: inner core tube
- 41: enlarged end
- 5: positioning ring
- 51: rod-shaped support member
- 52: hemline
- 53: autologous valve annulus adaption segment
- 54: atrial tissue adaption segment
- 55: skeleton
- 56: membrane
- 6: outer sheath tube
- 7: valve stent delivery apparatus
- 8: connection structure
- 9: securing clamp
- 10: valve clamp
- 101: atrial support segment
- 102: ventricular segment

### DETAILED DESCRIPTION

In the present application, "close end" refers to one end close to a surgical operator, and "far end" refers to one end far away from the surgical operator.

### Embodiment one

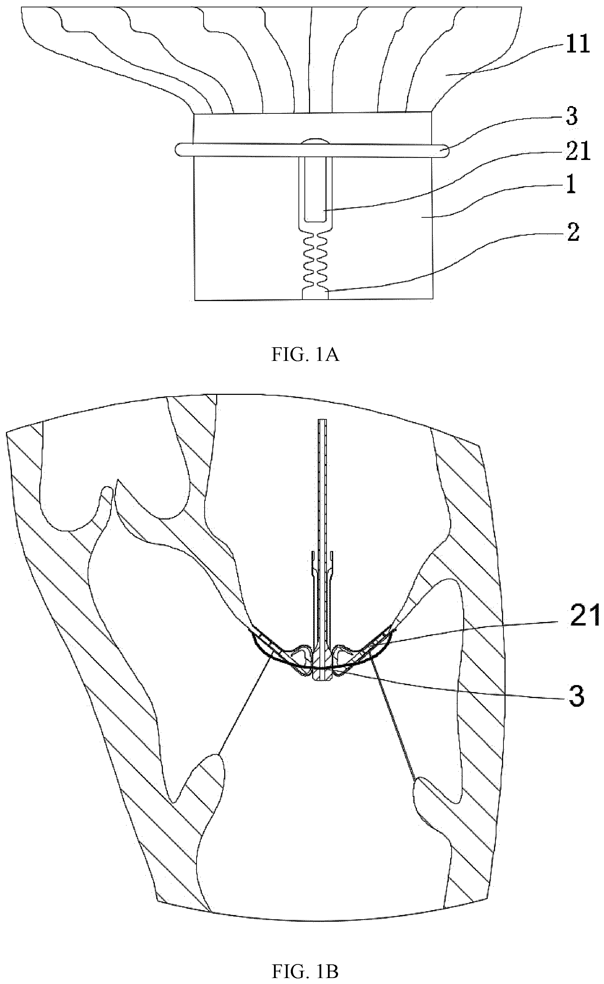

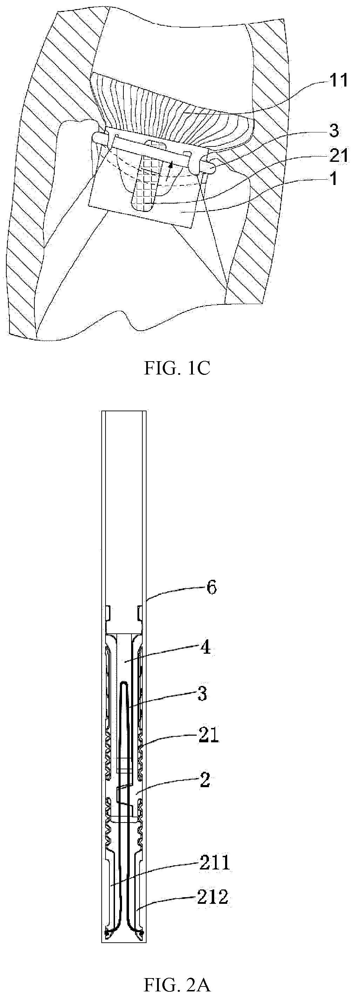

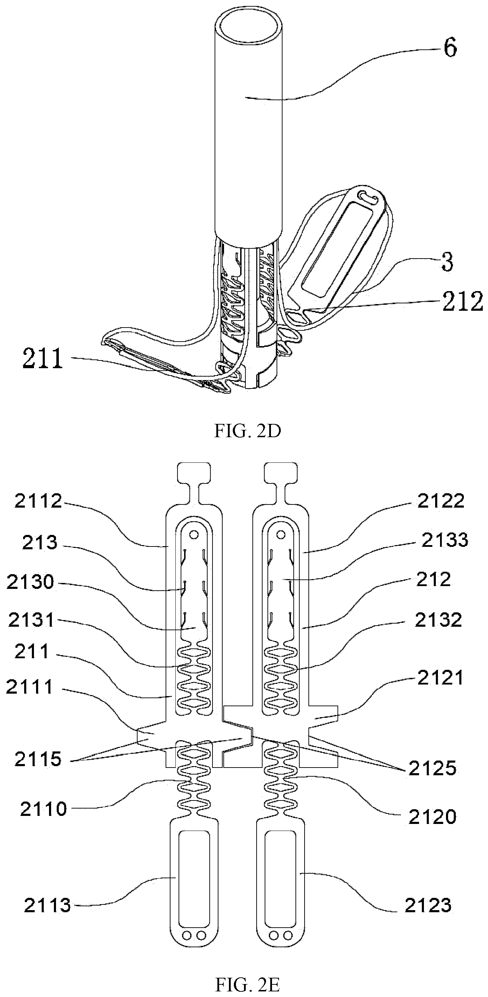

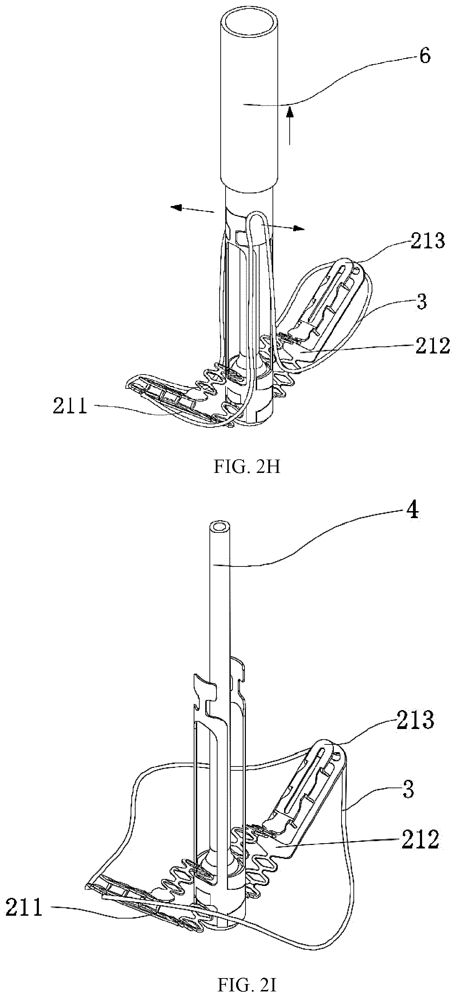

As illustratively shown in FIGS. 1A to 1C and 2A to 2J, according to an embodiment of the present application, a prosthetic valve replacement system is provided and may include a valve stent 1 and a valve clamping and securing apparatus 2 configured with the valve stent 1. The valve clamping and securing apparatus 2 may include a valve clamping mechanism 21 and a hoop member 3, and the hoop member 3 is disposed on the valve clamping mechanism 21, as shown in FIGS. 1A to 1B.

As shown in FIGS. 1A to 1C and 2A to 2J, the valve clamping mechanism 21 may be composed of two halves each made of a shape memory alloy, that is, a first clamping half part 211 and a second clamping half part 212. As shown in FIG. 2E, the first clamping half part 211 and the second clamping half part 212 are each integrally formed. Before surgery, the two halves may surround an inner core tube 4 (described in detail below) to form a substantially sleeve-shaped configuration sleeved on the inner core tube 4, and the two halves are tightly sleeved by an outer sheath tube 6 externally and is restrained inside the outer sheath tube 6, as shown in FIGS. 2A to 2B.

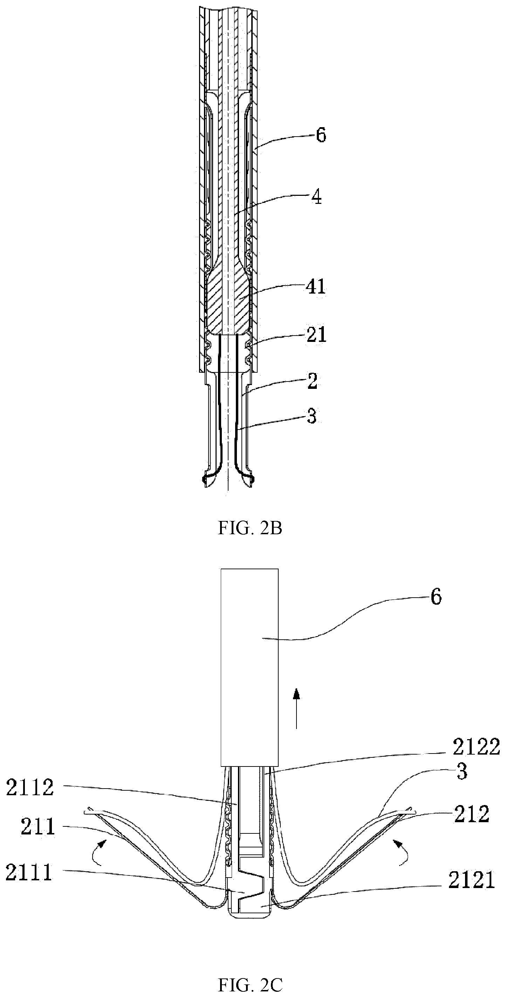

The first clamping half part 211 and the second clamping half part 212 may capture and clamp different autologous valve leaflets respectively, as shown in FIGS. 1B and 2C.

The first clamping half part 211 and the second clamping half part 212 are each provided with a tooth 213. Teeth 213 may grip the different autologous valve leaflets respectively and secure relative positions between the first clamping half part 211, the second clamping half part 212 and the different autologous valve leaflets, as shown in FIGS. 2E to 2G.

The teeth 213 may be tines, columns, barbs, or other structures that may secure the different valve leaflets respectively so that the first clamping half part 211 and the second clamping half part 212 can tightly grip the different autologous valve leaflets respectively.

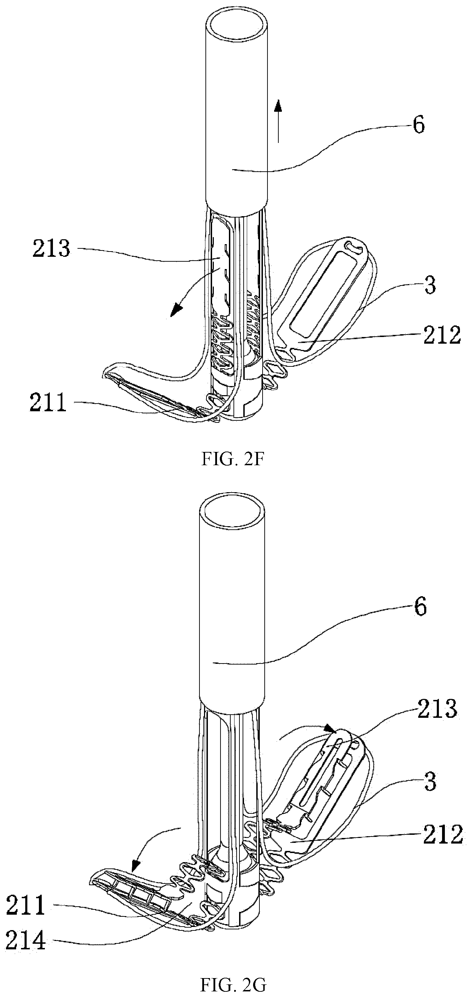

One end of the first clamping half part 211 and one end of the second clamping half part 212 are each provided with a valve leaflet receiving cavity 214. When the first clamping half part 211 and the second clamping half part 212 capture and clamp the different autologous valve leaflets respectively, portions of the different autologous valve leaflets are folded back inside the valve leaflet receiving cavities 214 respectively, as shown in FIGS. 1B and 2G.

More specifically, as shown in FIG. 2E, the first clamping half part 211 and the second clamping half part 212 may have complementary and mutually cooperative (such as embedding) shapes and form a detachable connection, for example, when a force is applied, or an enlarged end 41 of the inner core tube 4 is withdrawn from a cooperation portion, and the valve stent 1 is opened from the cooperation (such as embedding) portion, and may be separated from each other.

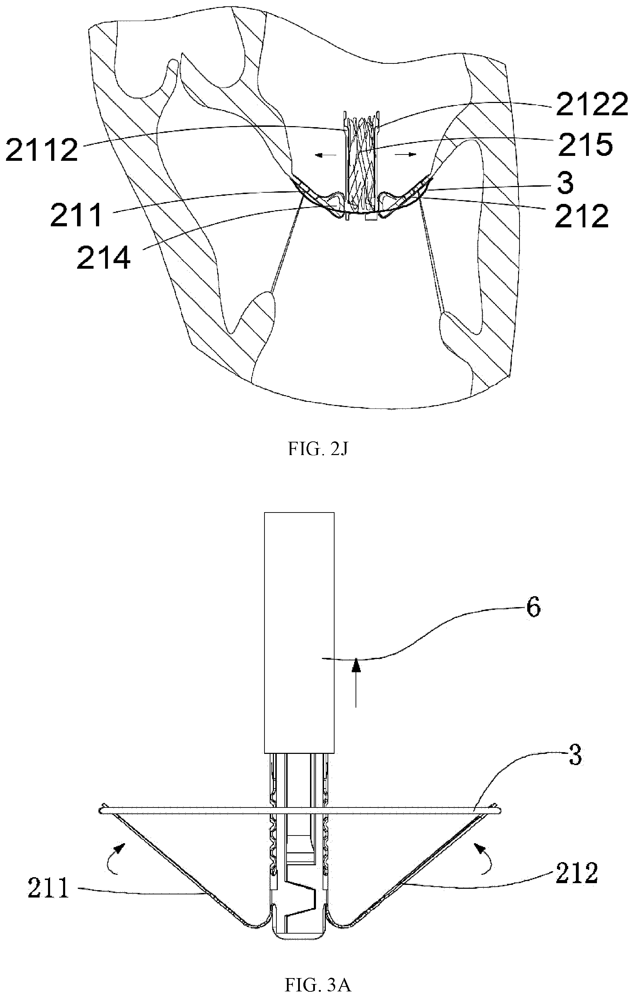

As shown in FIG. 2E, the first clamping half part 211 may have a first support segment 2112, a first detachable cooperation segment 2111 extending from and integrated with the first support segment 2112, and a first clamping segment 2113 further integrally extending downward from the first detachable cooperation segment 2111. A first clamping jaw 2130, which, for example, may have teeth 213, may be formed on the first support segment 2112, for example, by performing laser cutting on the main body of the first support segment 2112 and may be still integrally connected to the first support segment 2112 through a spring segment 2131. Similarly, the first clamping segment 2113 is integrally connected to the first detachable cooperation segment 2111 through a spring segment 2110. The first clamping half part 211 is integrally made of a memory alloy, and the spring segment 2131 and the spring segment 2110 each have a configuration of a rhombus wave, an S-shaped rod, or a thin and straight rod, as shown in the figures so that the first clamping half part 211 can be prone to resilient deformation, for example, be prone to be straight to be fitted into the outer sheath tube 6 before the surgery (for example, as shown in FIGS. 2B and 2E) and be bent to fold the valve leaflet back during and after the surgery (as shown in FIGS. 2J to 3D and described in detail below).

Similarly, as shown in FIG. 2E, the second clamping half part 212 may have a second support segment 2122, a second detachable cooperation segment 2121 extending from and integrated with the second support segment 2122, and a second clamping segment 2123 further integrally extending downward from the second detachable cooperation segment 2121. A second clamping jaw 2133, which, for example, may have teeth 213, may be formed on the second support segment 2122, for example, by performing laser cutting on the main body of the second support segment 2122 and may be still integrally connected to the second support segment 2122 through a spring segment 2132. Similarly, the second clamping segment 2123 is integrally connected to the second detachable cooperation segment 2121 through a spring segment 2120. The second clamping half part 212 is integrally made of a shape memory alloy, and the spring segment 2132 and the spring segment 2120 each have a configuration of a rhombus wave, an S-shaped rod, or a thin and straight rod, as shown in the figures so that the second clamping half part 212 can be prone to resilient deformation, for example, be prone to be straight to be fitted into the outer sheath tube 6 before the surgery (for example, as shown in FIGS. 2B and 2E) and be bent to fold the valve leaflet back during and after the surgery (as shown in FIGS. 2J to 3D and described in detail below).

As shown in FIG. 2B, the first clamping half part 211 is integrally formed by the first support segment 2112, the first detachable cooperation segment 2111 and the first clamping segment 2113, the second clamping half part 212 is integrally formed by the second support segment 2122, the second detachable cooperation segment 2121 and the second clamping segment 2123, and the first clamping half part 211 and the second clamping half part 212 are sleeved on the inner core tube 4 in a sleeve-shaped configuration and are put into the outer sheath tube 6. Two protrusions 2115 of the first detachable cooperation segment 2111 and two complementary-shaped recesses 2125 of the second detachable cooperation segment 2121 are correspondingly engaged (or embedded) with each other to secure and maintain the sleeve-shaped configuration formed by the first clamping half part 211 and the second clamping half part 212. In this case, the enlarged end 41 of the inner core tube 4 has penetrated into the sleeve formed by the first detachable cooperation segment 2111 and the second detachable cooperation segment 2121 (as shown in FIGS. 2A to 2B).

During the surgery, when the preceding valve clamping mechanism 21 fitted into the outer sheath tube 6 reaches the surgical position, the outer sheath tube 6 is withdrawn toward the close end (that is, in the direction of a straight line arrow as shown in FIG. 2C) to first expose (release) the first clamping segment 2113, the second clamping segment 2123, the first detachable cooperation segment 2111 and the second detachable cooperation segment 2121. At this time, the first clamping segment 2113 and the second clamping segment 2123 are exposed and released, and are bent upward (as shown by a curved arrow in FIG. 2C) toward the first and second support segments 2112 and 2122 respectively by use of their own shape memory effect (spring segments) from a previously upright state substantially in line with the first and second support segments 2112 and 2122, as shown in FIGS. 2C to 2D. During the surgery, under this state, the different autologous valve leaflets have been captured and attached to the first clamping segment 2113 and the second clamping segment 2123 respectively.

Afterward, the outer sheath tube 6 is further withdrawn to expose the first support segment 2112, the first clamping jaw 2130 on the first support segment 2112, the second support segment 2122 and the second clamping jaw 2133 on the second support segment 2122 (as shown in FIG. 2F). The first clamping jaw 2130 and the second clamping jaw 2133 are each bent downward (as shown by a curved arrow in FIG. 2F) through the spring segments by use of their own shape memory effect (the spring segments) to be pressed against the first clamping segment 2113 and the second clamping segment 2123 respectively from a previously embedded state in the first and second support segments 2112 and 2122 and complying to their overall shapes respectively, as shown in FIGS. 2H to 2I. During the surgery, under this state, the autologous valve leaflets previously captured and attached to the first clamping segment 2113 is clamped between the first clamping jaw 2130 and the first clamping segment 2113 and is gripped and secured by the teeth 213 on the first clamping jaw 2130; and the autologous valve leaflets previously captured and attached to the second clamping segment 2123 is clamped between the second clamping jaw 2133 and the second clamping segment 2123 and is gripped and secured by the tooth 213 on the second clamping jaw 2133, as shown in FIG. 2J.

At this time, the valve clamping mechanism 21 is in a capturing state, and an expandable passage 215 is formed between the first support segment 2112 and the second support segment 2122, as shown in FIG. 2J, so that the valve stent 1 which subsequently enters can enter and leave room for expansion.

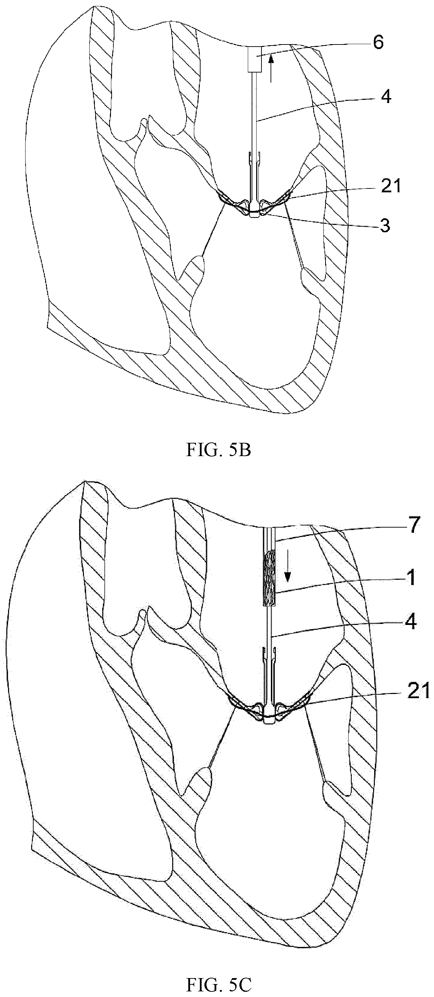

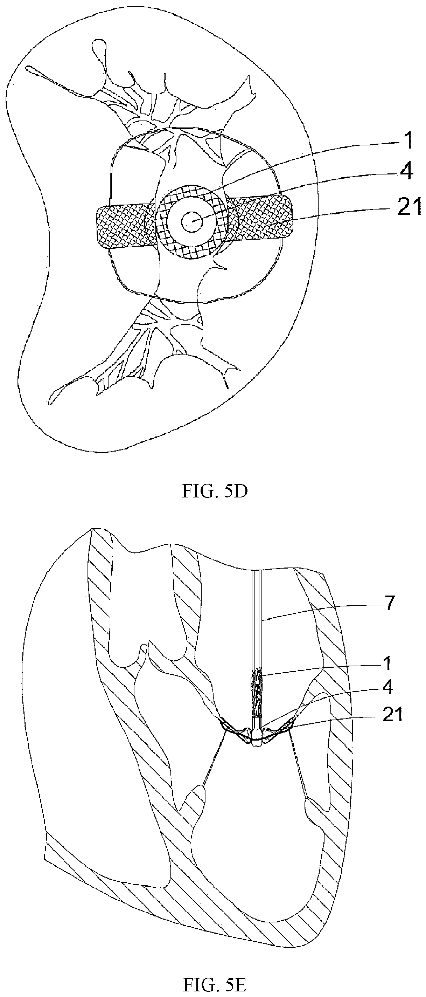

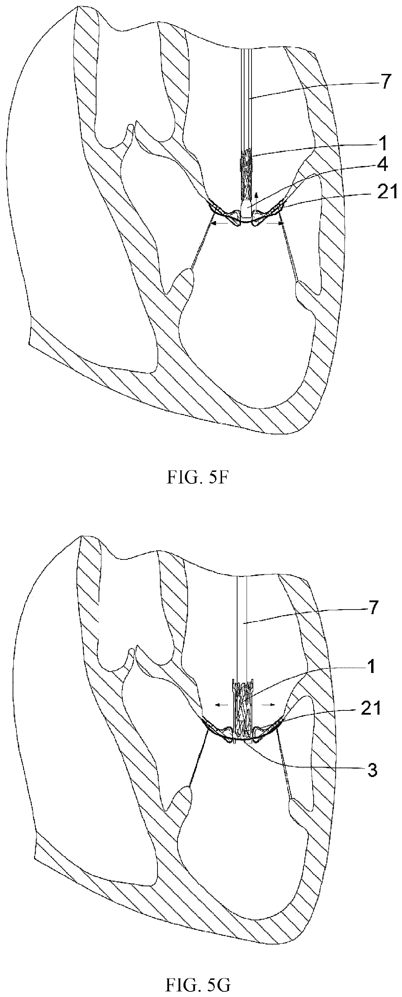

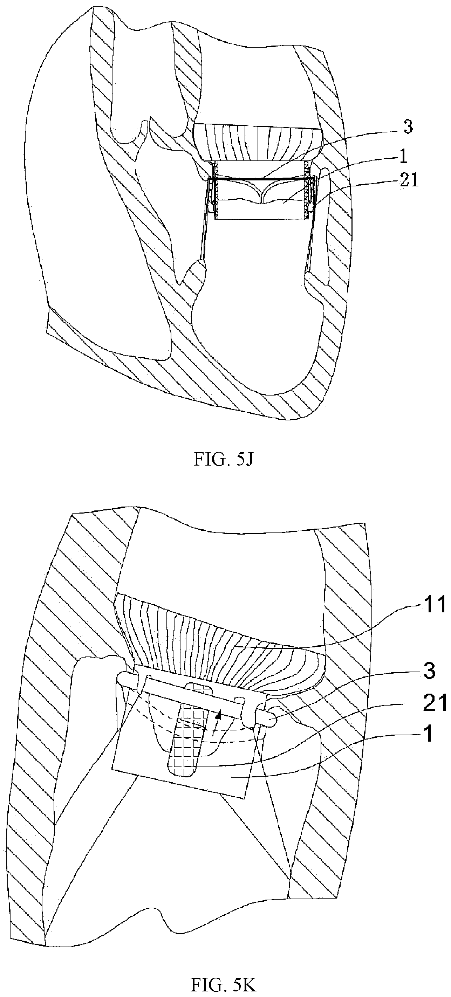

After the valve stent 1 is carried by a valve stent delivery apparatus 7 (such as FIG. 5C) along the path of the core inner tube 4 to enter to be sleeved on the first and second support segments 2112 and 2122 and enter the expandable passage 215 (as shown in FIG. 5E), the enlarged end 41 of the core inner tube 4 is withdrawn and separated from the valve clamping mechanism 21 (as shown in FIG. 5F), and the valve stent 1 may be released to expand (swell) radially to the functional form and enable the first clamping half part 211 to be further separated from the second clamping half part 212 (as shown in FIGS. 5G to 5H).

The hoop member 3 is generally composed of a linear or strip-shaped flexible member, for example, a closed ring or hoop formed by passing through holes at the ends of the first clamping segment 2113 and the second clamping segment 2123 (as shown in FIG. 2C to 2D) and capable of restraining the positions of the first clamping segment 2113 and the second clamping segment 2123 after release (that is, radially expanded).

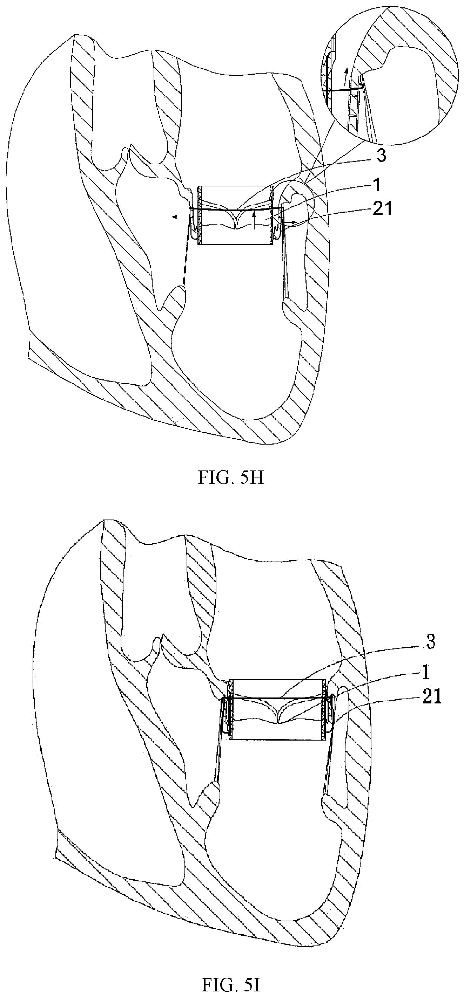

The perimeter of the hoop member 3 in a fully unfolded state is generally disposed to be less than the circumference of the cross-sectional circle of the fully expanded (swollen) valve stent 1. In this way, after radially expanded (swollen), the valve stent 1 may be ensured to be tightly hooped with a certain pre-stress by the hoop member 3 (at this time, the hoop member 3 also clamps the autologous valve annulus tightly in the middle, as shown in FIGS. 2J and 5I to 5J), and the valve stent 1 is restrained so as to avoid damage caused by excessive expansion of the valve stent 1 beyond the size of the autologous valve annulus. For another aspect, in the process of radially expanding the valve stent 1 in position to restore the functional form, since the valve stent 1 is tightly hooped by the hoop member 3 fastened to the valve clamping mechanism 21 clamping the autologous valve leaflets, the valve stent 1 is radially supported by the hoop member 3 so that the valve stent 1 can be indirectly secured to autologous valve leaflet tissue through the hoop member 3.

The hoop member 3 may be a flexible ring or hoop integrally, for example, a ring or hoop that may be composed of a substantially flexible bar-shaped, filament-shaped, linear-shaped, stranded wire or rope-shaped member. The outer periphery of the hoop member 3 may also be coated, for example, with a membrane that may increase the friction force with the autologous valve leaflets.

The hoop member 3 may be a closed-loop structure, or the hoop member 3 and the valve clamping mechanism 21 cooperate with each other to form a closed-loop structure. After the valve clamping mechanism 21 captures the autologous valve leaflets, the hoop member 3 is at the outer periphery of the autologous valve leaflets and encircles the autologous valve leaflets (as shown in FIGS. 2J and 5I to 5J).

The valve stent 1 may also be, for example, a self-expandable reticulated stent in the shape of, for example, a sleeve-shaped configuration that is expandable and secured in position when the valve stent 1 reaches the surgical site.

A positioning ring 11 positioned within the atrium during the surgery is also disposed on the valve stent 1 and is configured to have a form that adapts to the physiological structure of the autologous valve annulus. A membrane may be coated on the positioning ring 11 to avoid perivalvular leakage.

To sum up, the valve clamping and securing apparatus 2 may present a first form and a second form.

When the valve clamping and securing apparatus 2 is in the first form, the valve clamping mechanism 21 is configured to be pre-secured to, for example, the middle region of the autologous valve in a manner of locally clamping the autologous valve leaflets, and at this time, the hoop member 3 is in a relaxing form in which the valve stent 1 is not hooped. At this time, the autologous valve leaflets may maintain the function of locally opening and closing.

When the valve clamping and securing apparatus 2 is in the second form, the valve stent 1 is radially expanded and drives the valve clamping mechanism 21 and the hoop member 3 to be expanded synchronously and to be tightly hooped finally by the hoop member 3. During this process, the hoop member 3 is located between the autologous valve leaflets and the chordae tendineae tissue, is gradually lifted upward (for example, as shown by an upward arrow in FIG. 5H), hoops the autologous valve leaflets and adjacent tissue and drives the clamped autologous valve leaflets to be lifted upward and gradually folded back.

According to an embodiment, when the valve stent 1 is radially expanded and drives the valve clamping mechanism 21 and the hoop member 3 to be unfolded synchronously, the hoop member 3 pulls the hooped autologous valve leaflets and chordae tendineae upward to move toward the directions of connecting points of the hoop member 3 and the valve clamping mechanism 21 and drives the autologous valve leaflets to be folded back; and finally, the hoop member 3 secures the portion of the autologous valve leaflets, the chordae tendineae and the valve clamping mechanism 21 around the outer periphery of the valve stent 1 circumferentially, for example, as shown in FIG. 1C.

According to an embodiment, when capturing and clamping the autologous valve leaflets, the valve clamping mechanism 21 moves toward the direction of the atrium; and when the valve stent 1 is radially expanded to restore a preset form, the valve stent 1 and the hoop member 3 cooperate with each other, and the portions of the autologous valve leaflets are lifted upward while being folded back so that the position of the valve leaflet can be raised to avoid blocking the left ventricular outflow tract. In contrast, due to the relatively long lengths of anterior autologous valve leaflets of a mitral valve, a traditional replacement surgery can make the blockage of the left ventricular outflow tract by the anterior autologous valve leaflet very evident. Adoption of the present solution can effectively reduce the impact of the original autologous valve leaflet tissue on blood flow after the replacement, because the autologous valve leaflet tissue is not only partially folded back to mitigate the risk of flow blocking but also lifted upward by use of the radial expansion of the valve stent 1 to reduce the possibility of blocking the blood flow of the left ventricular outflow tract by the autologous valve leaflet tissue.

Operation of the prosthetic valve replacement system is described below.

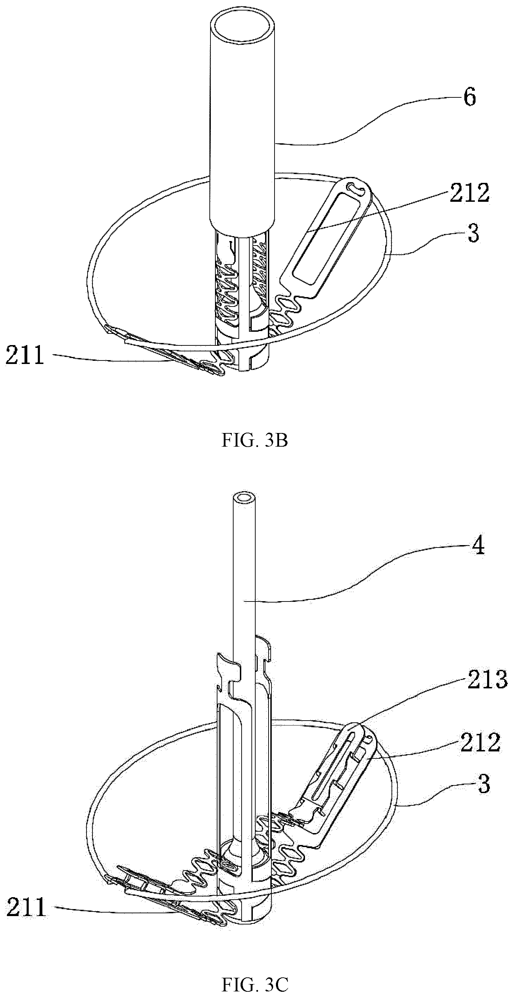

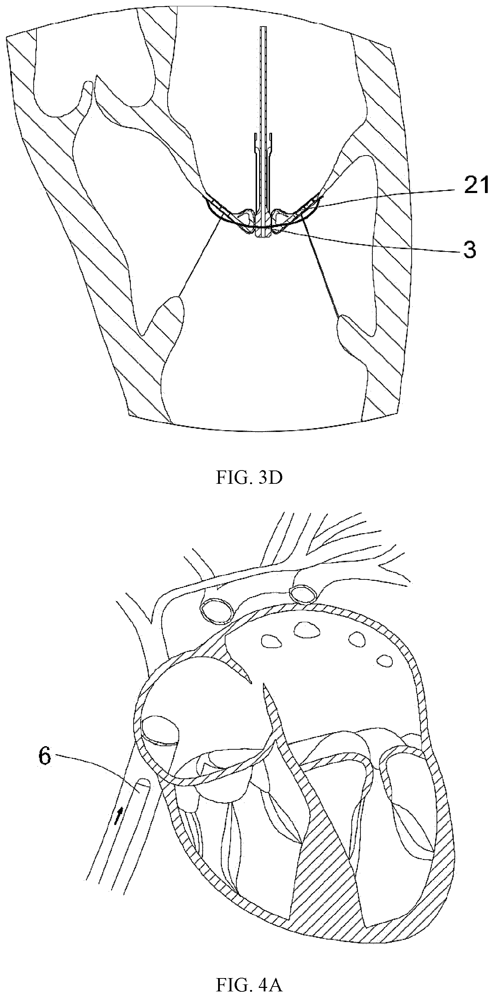

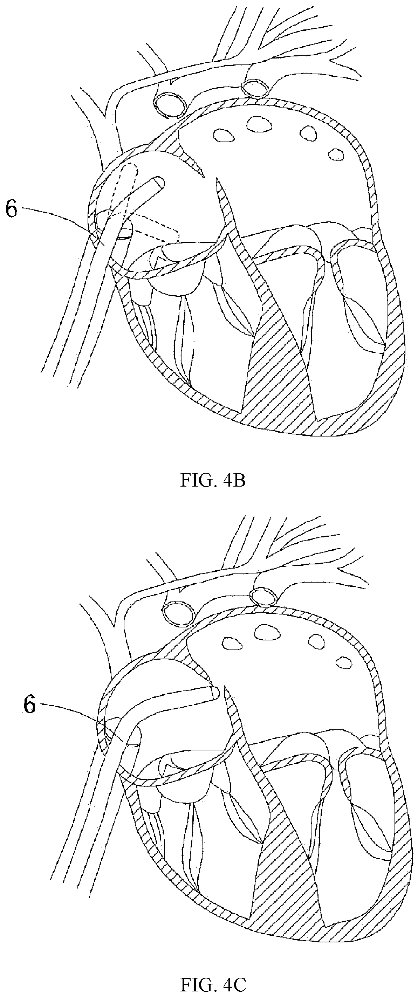

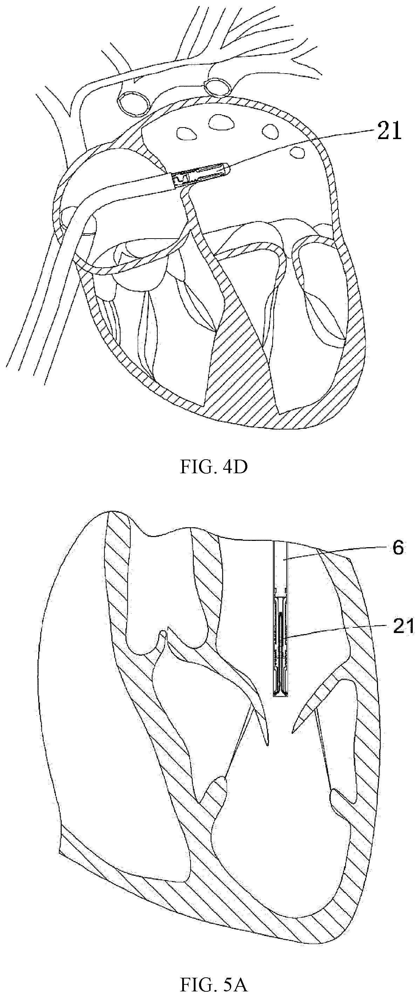

An exemplary operation process for repairing the mitral valve in embodiment one is described below.
1. The outer sheath tube 6 is operated to enter the heart from the inferior vena cava, and the outer sheath tube 6 is subsequently operated to enable the valve clamping mechanism 21 to pass through the interatrial septum, as shown in FIGS. 4A to 4D; and the outer sheath tube 6 is continuously operated to be bent to enable the valve clamping mechanism 21 to face the orifice of the mitral valve.
2. The outer sheath tube 6 is operated to enable the first clamping half part 211 and the second clamping half part 212 of the valve clamping mechanism 21 to capture the anterior leaflet and the posterior leaflet of the mitral valve respectively and clamp the corresponding anterior leaflet and posterior leaflet together with the first clamping jaw 2130 and the second clamping jaw 2133 respectively, as shown in FIGS. 5A to 5B, and the specific steps are shown and described with reference to, for example, FIGS. 2A to 2G; and after the valve clamping mechanism 21 clamps the valve leaflet, the outer sheath tube 6 is withdrawn from the body, and the inner core tube 4 is left in the original position.
3. Afterward, the valve stent delivery apparatus 7 is operated to enter the heart along the path of the inner core tube 4, as shown in FIGS. 5C to 5E; when the valve stent 1 enters the middle region of the valve clamping mechanism 21, the inner core tube 4 is operated (for example, is withdrawn) to be detached and separated from the valve clamping mechanism 21, as shown in FIGS. 5F and 5G; and afterward, the valve stent delivery apparatus 7 is further operated to gradually release the valve stent 1, and the valve stent 1 is radially expanded to restore the functional form.
4. The radial expansion of the valve stent 1 enables the first clamping half part 211 and the second clamping half part 212 of the valve clamping mechanism 21 to move further in the direction, for example, as shown by the curved arrow in FIG. 3A, to close to or even abut onto the outer periphery of the valve stent 1, and thereby the clamped valve leaflet is partially folded back; and the further radial expansion of the valve stent 1 enables the valve stent 1 to be expanded outwardly, be supported on the hoop member 3 tightly hooped at the outer periphery of the valve stent 1, restore the functional form and thereby be anchored in the heart, and at this time, the autologous valve leaflets are clamped between the hoop member 3 and the valve stent 1, after which the valve stent delivery apparatus 7 may be withdrawn from the body, as shown in FIGS. 5H to 5K.

### Embodiment two

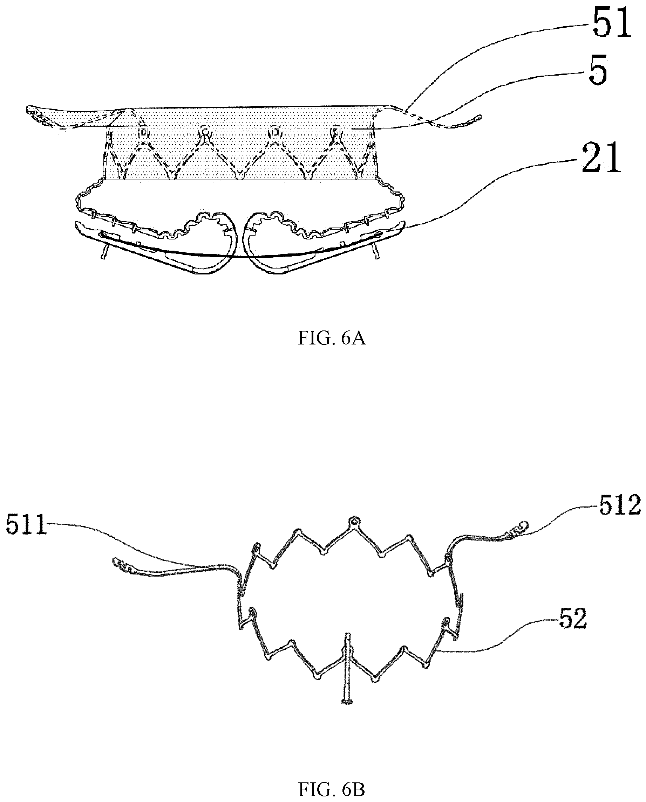

Embodiment two is substantially the same as embodiment one, except that the valve clamping mechanism 21 in this embodiment further includes a positioning ring 5. The positioning ring 5 may supply an anchoring force to the valve clamping mechanism 21 at the valve annulus or the atrium so that after the first clamping half part 211 and the second clamping half part 212 have captured and clamped the different valve leaflets, and when the valve stent 1 has not entered the release position, the vale clamping mechanism 21 can be well maintained at the position within the heart, preventing the valve clamping mechanism 21 from slipping, as shown in FIGS. 6A to 6C.

In this embodiment, the valve clamping and securing apparatus 2 may include the valve clamping mechanism 21 and the hoop member 3, and the hoop member 3 may be disposed on the valve clamping mechanism 21. In this embodiment, the positioning ring 5 is also provided and may be configured to have the form that adapts to the physiological structure of the valve annulus. The valve clamping and securing apparatus 2 may have the first form and the second form. When the valve clamping and securing apparatus 2 is in the first form, the valve clamping mechanism 2 may be configured to be pre-secured to, for example, the middle region of the autologous valve annulus in a manner of locally clamping the autologous valve leaflets, the hoop member 3 may be in a relaxing form, the autologous valve leaflets can maintain the function of locally opening and closing, and the positioning ring 5 is located within the atrium; and when the valve clamping and securing apparatus 2 is in the second form, the valve stent 1 may be radially expanded and drive the valve clamping mechanism 21 and the hoop member 3 to be unfolded synchronously, the hoop member 3 may gradually lift the autologous valve leaflets and the adjacent tissue, and the autologous valve leaflets are folded back finally.

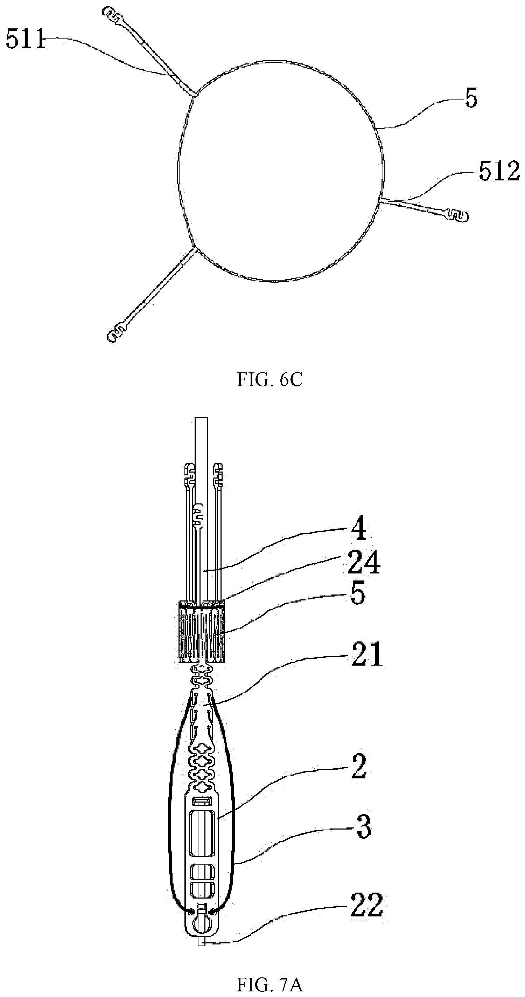

More specifically, in this embodiment, the positioning ring 5 may be composed of a radial rod-shaped support member 51. The rod-shaped support member 51 may effectively prevent the valve stent from shaking left and right and help reduce or avoid the occurrence of perivalvular leakage, as shown in FIGS. 6A to 6C. The rod-shaped support member 51 may include two main support members 511 and at least one auxiliary support member 512. One end of the rod-shaped support member 51 may be securely connected to the body of the positioning ring as a securing end, and the other end of the rod-shaped support member 51 is free as a free end. For example, a flexible hemline 52 may be disposed between adjacent rod-shaped support members 51. When fully released, the positioning ring 5 can be self-adapted to a closed-loop structure such as a D-shaped structure. A connecting line of free ends of the two main support members 511 can form a straight line segment for supplying the D-shaped structure. A free end of an auxiliary support member 512 may be located on an arc line segment of the D-shaped structure, for example, as shown in FIG. 6C.

In this embodiment, the prosthetic valve replacement system may further include a capturing controller 22, a detachable controller 23 and a positioning ring controller 24. The detachable controller 23 may control the detachment and separation of the first clamping half part 211 and the second clamping half part 212.

In this embodiment, during pre-mounting, the first clamping half part 211 and the second clamping half part 212 are fitted into the outer sheath tube 6 in a mutually engaged and straightened form; when the valve clamping and securing apparatus 2 is in, for example, the middle region of the autologous valve and needs to capture the autologous valve leaflets, the outer sheath of the outer sheath tube 6 is withdrawn toward the close end; and the capturing controller 2 is further operated so that the far end of the first clamping half part 211 and the far end of the second clamping half part 212 can be released to restore the preset form, and the valve clamping and securing apparatus 2 can be in the first form.

In this embodiment, when the first clamping half part 211 and the second clamping half part 212 capture and clamp the different autologous valve leaflets respectively, the outer sheath of the outer sheath tube 6 continues to be withdrawn toward the close end so that the valve clamping and securing apparatus 2 can be fully exposed, the positioning ring controller 24 is operated so that the positioning ring 5 can be released to restore the preset form, and the positioning ring 5 may conform to the uneven contour of the patient's own valve annulus and does not restrict the contractile function of the atrium.

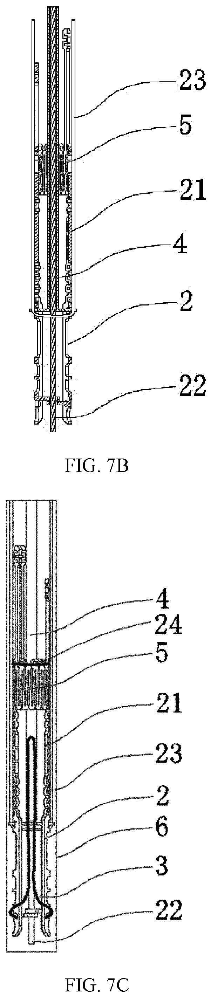

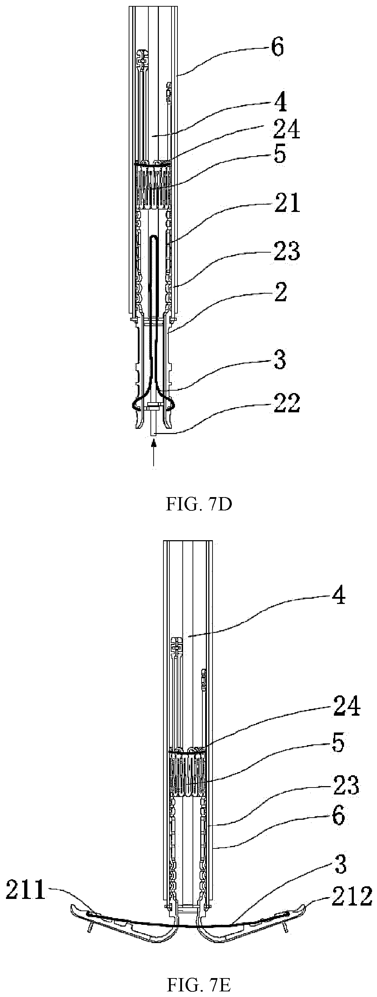

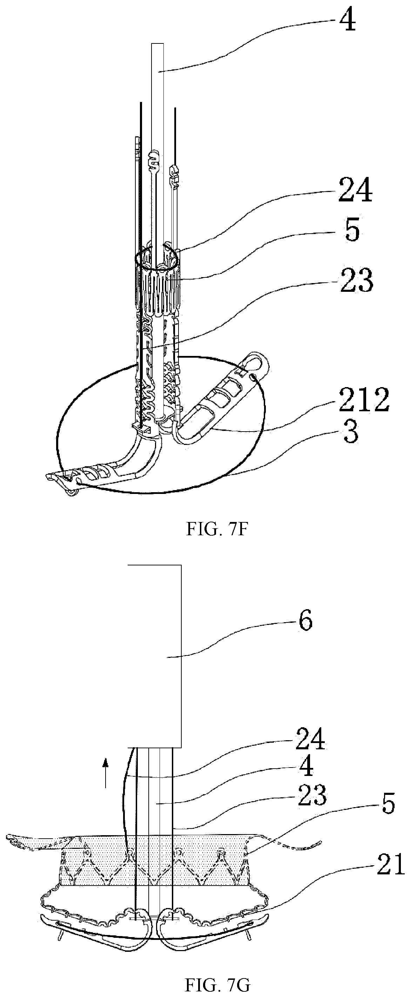

In this embodiment, the first clamping half part 211 and the second clamping half part 212 may be two separate members and are securely connected to the positioning ring 5, as shown in FIG. 7E, and the first clamping half part 211 and the second clamping half part 212 are each made of, for example, a metal memory alloy material such as nitinol.

In this embodiment, the positioning ring 5 has a preset form and may be in a grid-shaped, "Z"-shaped, or waved form, as shown in FIG. 6B. This configuration enables the positioning ring 5 to undergo large-scale size adjustments while the positioning ring 5 is still in a range of resilient deformation.

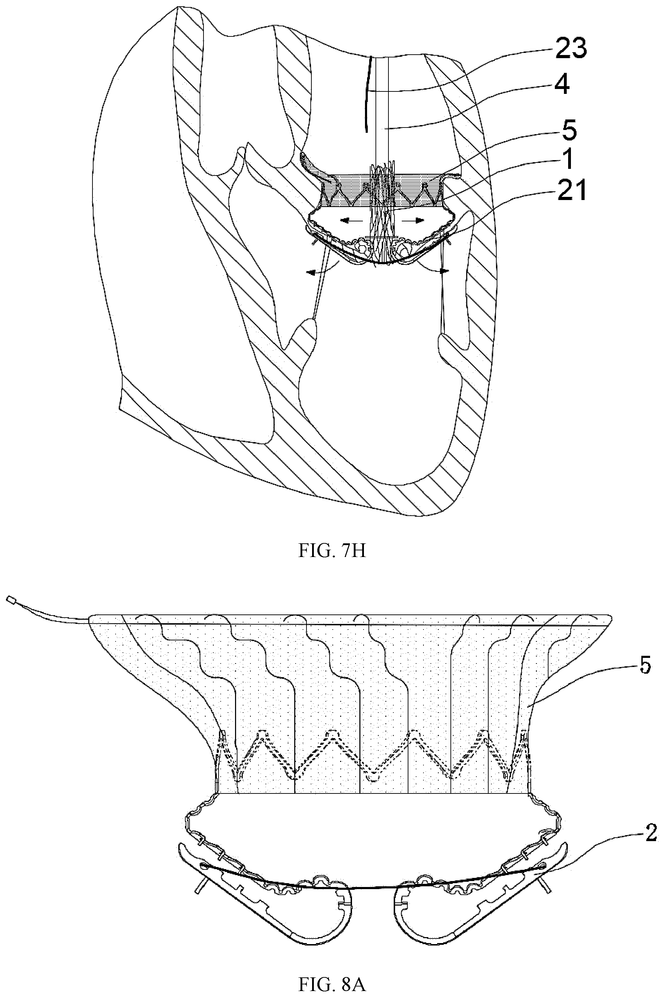

In this embodiment, the positioning ring 5 and the valve clamping mechanism 21 may have an integral structure, as shown in FIG. 8A, or may also have separate structures.

In this embodiment, the positioning ring 5 may be made of a metal memory alloy material such as nitinol, and the positioning ring 5 may be coated with a membrane.

An exemplary operation process for repairing the mitral valve in embodiment two is described below.
1. The outer sheath tube 6 is operated to enter the heart from the inferior vena cava, and the outer sheath tube 6 is subsequently operated to enable the valve clamping mechanism 21 to pass through the interatrial septum, as shown in FIGS. 4A to 4D; and the outer sheath tube 6 is continuously operated to be bent to enable the valve clamping mechanism 21 to face the orifice of the mitral valve.
2. The outer sheath of the outer sheath tube 6 is operated to be withdrawn toward the close end; and the capturing controller 2 may be further operated so that the far end of the first clamping half part 211 and the far end of the second clamping half part 212 can be released to restore the preset form and can capture and clamp the anterior leaflet and the posterior leaflet of the mitral valve respectively; when the valve clamping mechanism 21 clamps the valve leaflet, the outer sheath of the outer sheath tube 6 continues to be withdrawn toward the close end so that the valve clamping and securing apparatus 2 can be fully exposed; the positioning ring controller 24 is operated so that the positioning ring 5 can be released to restore the preset form, the outer sheath tube 6 is withdrawn from the body, and the inner core tube 4 is left in the original position, as shown in FIGS. 7C to 7G.
3. The valve stent delivery apparatus 7 is operated to enter the heart along the path of the inner core tube 4; when the valve stent 1 enters the middle region of the valve clamping mechanism 21, the detachable controller 23 is operated so that the first clamping half part 211 and the second clamping half part 212 can be separated from each other, as shown in FIG. 7H; and the valve stent delivery apparatus 7 is further operated to gradually release the valve stent 1, and the valve stent 1 is radially expanded to restore the preset form.
4. The radial expansion of the valve stent 1 enables the first clamping half part 211 and the second clamping half part 212 of the valve clamping mechanism 21 to abut onto the outer periphery of the valve stent 1, the valve stent 1 is further radially expanded to support the hoop member 3 and restore the preset form to be anchored in the heart, the autologous valve leaflets are clamped between the hoop member 3 and the valve stent 1, and afterward, the valve stent delivery apparatus 7 is withdrawn from the body.

In this regard, related configurations and ideas of embodiment two are similar to those of embodiment one, so details are not repeated herein.

### Embodiment three

Embodiment three is substantially the same as embodiment one, except that the valve stent 1 in this embodiment is a balloon-expandable stent.

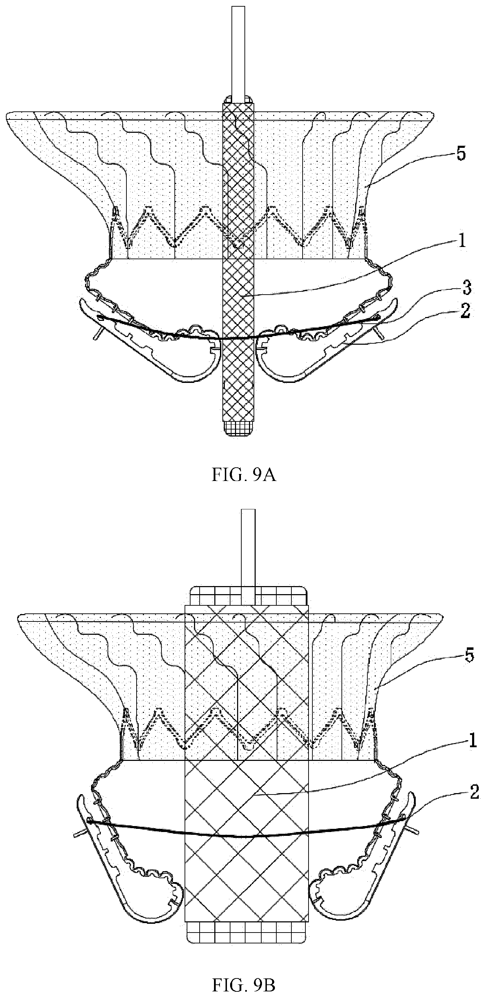

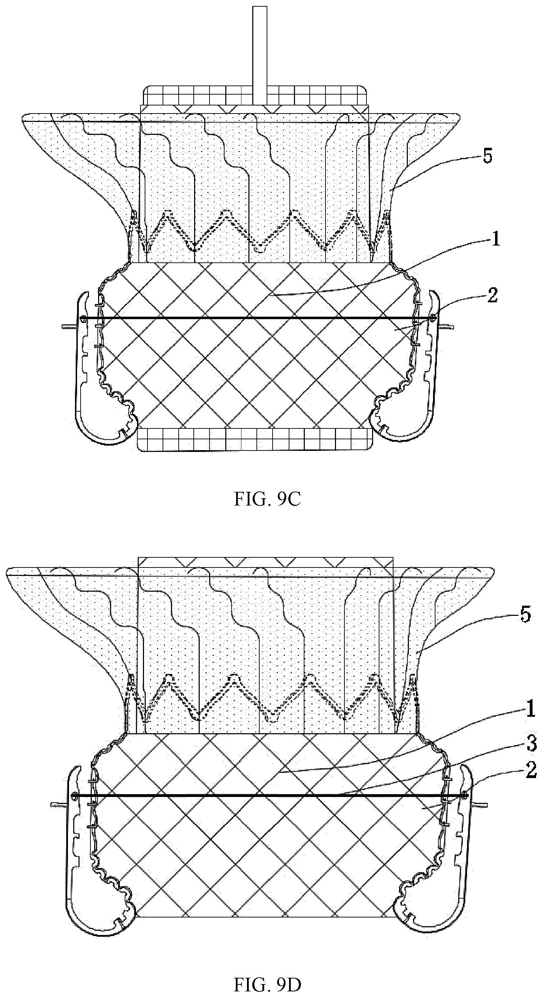

In this embodiment, the valve stent 1 may be a balloon-expandable valve, as shown in FIGS. 9A to 9D. When capturing and clamping the autologous valve leaflets, the valve clamping mechanism 21 moves toward the direction of the atrium. When the valve stent 1 is in, for example, the middle region of the valve clamping mechanism 21, the valve stent 1 may be released and radially expanded to restore the functional form, and the valve stent 1 and the hoop member 3 may cooperate with each other, fold the portion of the autologous valve leaflets back gradually and incrementally (as shown in FIGS. 3D and 5B to 5J) and drive the autologous valve leaflets to be upward lifted and folded back while being lifted upward (for example, as shown by an upward arrow in FIG. 5H) so that the left ventricular outflow tract can be further widened, and the risk of blocking the left ventricular outflow tract by the autologous valve leaflets can be reduced. In contrast, due to the relatively long length of the anterior autologous valve leaflet of the mitral valve, the traditional replacement surgery can make the blockage of the left ventricular outflow tract by the anterior autologous valve leaflet very evident, and the axial height of the balloon-expandable stent is shorter, and after the stent is implanted, the impact on the blood flow of the left ventricular outflow tract can be further reduced. The inventive idea and technical effect of the present application apparently solve the long-standing technical problems in the industry.

**In** this regard, related configurations and ideas of embodiment three are similar to those of embodiment one, so details are not repeated herein.

### Embodiment four

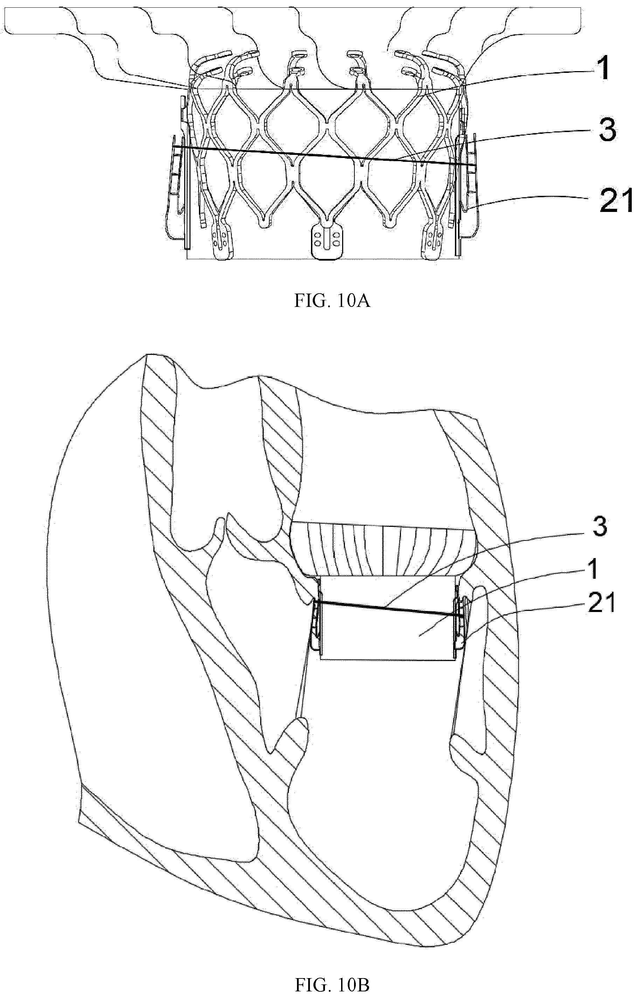

Embodiment four is substantially the same as embodiment three, except that connecting points of the hoop member 3 and the valve clamping mechanism 21 have different heights in this embodiment so that the hoop member 3 can selectively lift the autologous valve leaflets, as shown in FIGS. 10 A and 10B.

In this embodiment, the replacement system is configured to replace the mitral valve, the connecting points of the hoop member 3 and the valve clamping mechanism 21 have different heights. Specifically, a connecting point of the hoop member 3 and the valve clamping mechanism 21 in the anterior valve leaflet region is higher than a connecting point of the hoop member 3 and the valve clamping mechanism 21 in the posterior valve leaflet region. This design has the following advantages. According to the physiological anatomical structure, the length of the anterior valve leaflet is relatively long, and after the replacement, the anterior valve leaflet tends to block the left ventricular outflow tract, so the connecting position of the hoop member 3 and the valve clamping mechanism 21 in the anterior valve leaflet region is relatively high. In the second form, the hoop member 3 has a larger lifting amplitude to the anterior valve leaflet, ensuring that the anterior valve leaflet cannot affect the left ventricular outflow tract, while the hoop member 3 has a relatively small pulling amplitude in the posterior valve leaflet region, preventing the hoop member 3 from excessively pulling the chordae tendineae tissue and facilitating tissue protection.

In this regard, related configurations and ideas of embodiment four are similar to those of embodiment one, so details are not repeated herein.

### Embodiment five

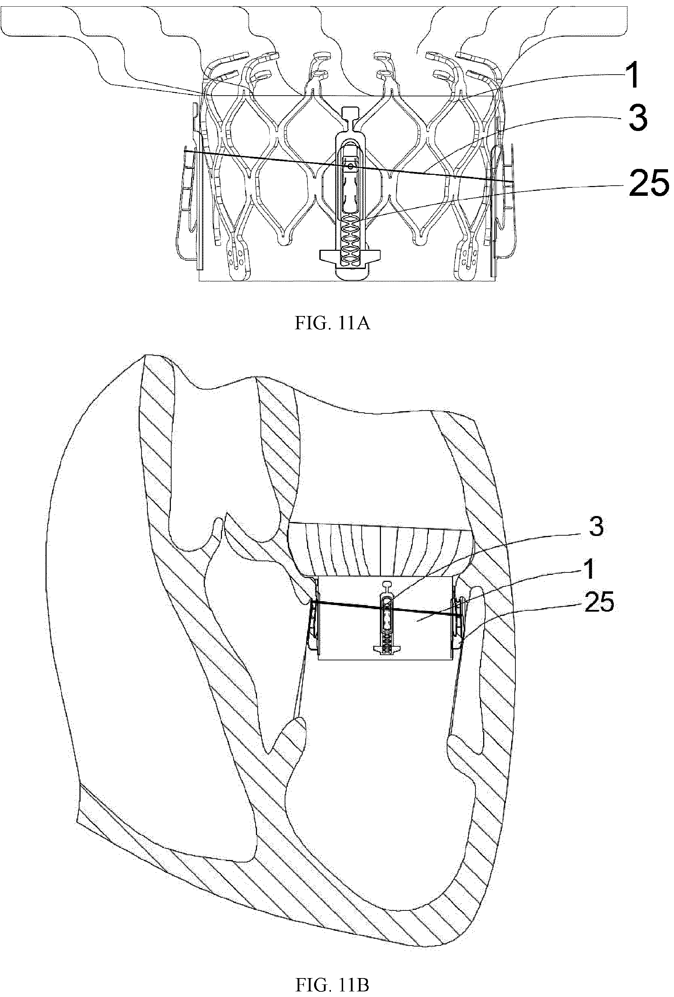

Embodiment five is substantially the same as embodiment one, except that the valve clamping mechanism 21 includes multiple clamps 25 and that connecting points of the hoop member 3 and the multiple clamps 25 have different heights in this embodiment, as shown in FIGS. 11A and 11B so that the hoop member 3 have a waved form in the second form.

In this embodiment, the valve clamping mechanism 21 includes the multiple clamps 25, and the connecting points of the hoop member 3 and the multiple clamps 25 have different heights so that the hoop member 3 have a waved form in the second form.

In this embodiment, a connecting position of a clamp 25 and the hoop member 3 in the anterior valve leaflet region needs to be higher than a connecting position of a clamp 25 and the hoop member 3 in the posterior valve leaflet region. This design has the following objects. According to the physiological anatomical structure, the length of the anterior valve leaflet is relatively long, and after the replacement, the anterior valve leaflet tends to block the left ventricular outflow tract, so the connecting position of the hoop member 3 and the valve clamping mechanism 21 in the anterior valve leaflet region is relatively high. In the second form, the hoop member 3 has a larger lifting amplitude to the anterior valve leaflet, ensuring that the anterior valve leaflet cannot affect the left ventricular outflow tract, while the hoop member 3 has a relatively small pulling amplitude in the posterior valve leaflet region, preventing the hoop member 3 from excessively pulling the chordae tendineae tissue and facilitating the tissue protection.

In this regard, related configurations and ideas of embodiment five are similar to those of embodiment one, so details are not repeated herein.

### Embodiment six

Embodiment six is substantially the same as embodiment one, except that a securing clamp 9 is disposed on the valve stent 1 in this embodiment. The securing clamp 9 is configured to clamp the autologous valve leaflets and increase the degree of abutting between the valve leaflet and the valve stent 1 while supplying a certain anchoring function, thereby supplying a good leak-proof function.

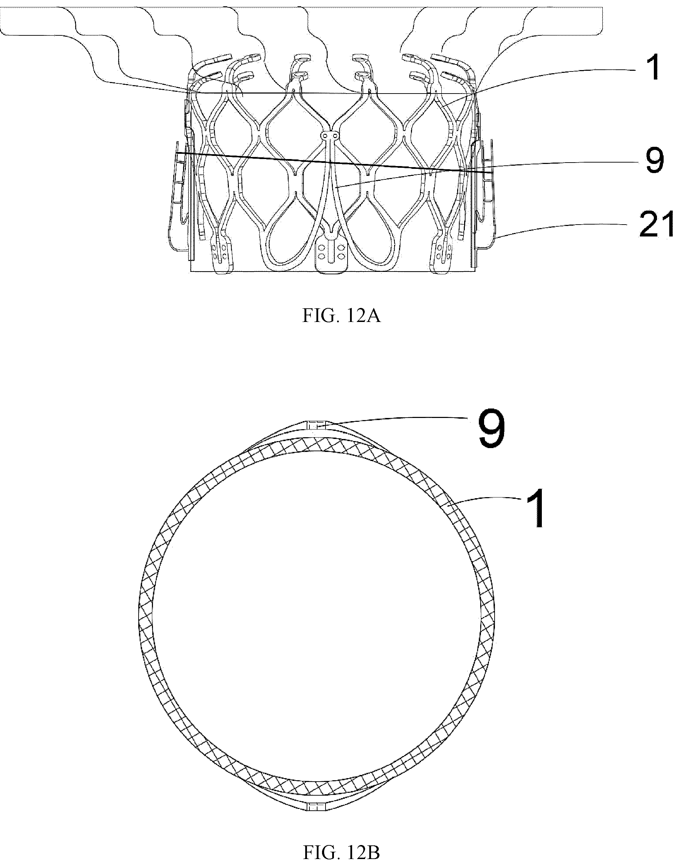

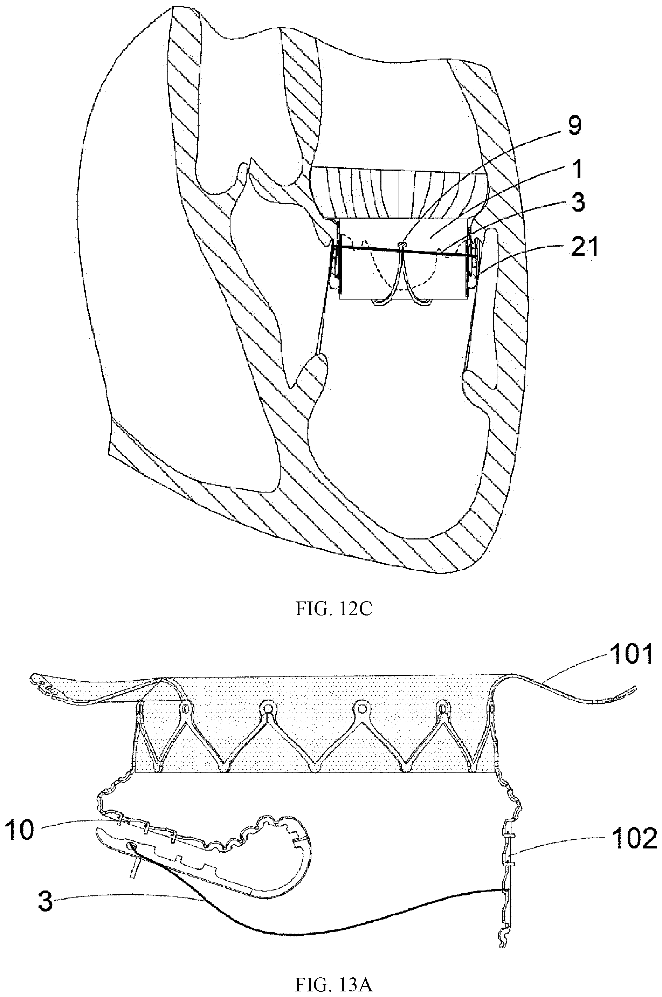

In this embodiment, the securing clamp 9 is disposed on the valve stent 1, and under the second form, the securing clamp 9 clamps the autologous valve leaflets and enables the autologous valve leaflets to be clamped between the valve stent 1 and the securing clamp 9, as shown in FIGS. 12A to 12C.

In this embodiment, the position of the securing clamp 9 does not overlap the position of the valve clamping mechanism 21.

In this regard, related configurations and ideas of embodiment six are similar to those of embodiment one, so details are not repeated herein.

### Embodiment seven

Embodiment seven is substantially the same as embodiment one, except that in this embodiment, the valve clamping and securing apparatus 2 only has a single valve clamp 10 and that one side of the hoop member 3 is connected to the single valve clamp 10, and the other side of the hoop member 3 is connected to a ventricular segment 102.

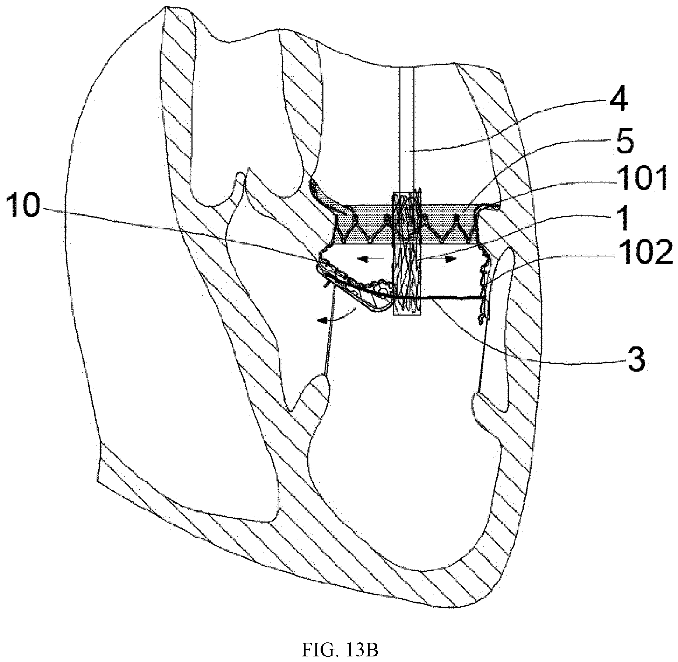

In this embodiment, the valve clamping and securing apparatus 2 includes the single valve clamp 10, an atrial support segment 101, the ventricular segment 102 and the hoop member 3. The hoop member 3 is connected to the single valve clamp 10 and the ventricular segment 102. The hoop member 3 has an inclined form on a cross section parallel to the central axis of the valve clamping and securing apparatus 2. The atrial support segment 101 is located inside the atrium and supplies a support force to the valve clamping and securing apparatus 2. The single valve clamp 10 is connected to the ventricular segment 102 and is located on one side of the anterior valve leaflet. The hoop member 3 is connected to the single valve clamp 10 and is securely connected to the ventricular segment 102 located in the posterior valve leaflet region so that the hoop member 3 can have an inclined form on the cross section parallel to the central axis of the valve clamping and securing apparatus 2, as shown in FIGS. 13A and 13B.

In this embodiment, the single valve clamp 10 is configured to clamp the anterior valve leaflet, and the height of the hoop member 3 in the region of the single valve clamp 10 needs to be higher than the height of the hoop member 3 in the posterior valve leaflet region. This design has the following objects. After the valve clamping and securing apparatus 2 is mounted in position, the anterior valve leaflet and the related chordae tendineae tissue are selectively lifted upward to avoid the impact on the left ventricular outflow tract, and the hoop member 3 does not pull the chordae tendineae and the posterior valve leaflet in the posterior valve leaflet region evidently, reducing the impact on the original tissue.

In this regard, related configurations and ideas of embodiment seven are similar to those of embodiment one, so details are not repeated herein.

The preceding description of example embodiments of the present application is provided for the purpose of illustration. The preceding description is not intended to be exhaustive or to limit the present application to the precise arrangements and/or configurations disclosed. Apparently, many modifications and variations may be made by those skilled in the art in light of the preceding teachings without departing from the present application. The scope of protection is as defined in the appended claims.

## Claims

1. A prosthetic valve replacement system, comprising a valve stent (1) and a valve clamping and securing apparatus (2) cooperating with the valve stent (1), wherein the valve clamping and securing apparatus (2) is constructed as a valve clamping mechanism (21) and a hoop member (3) for clamping autologous valve leaflets, and the hoop member (3) is at least partially disposed on the valve clamping mechanism (21),
wherein the valve clamping and securing apparatus (2) is configured to be able to present a first form and a second form, and the second form is present after the first form,
wherein when the valve clamping and securing apparatus (2) is in the first form, the valve clamping mechanism (21) is configured to be able to capture and clamp the autologous valve leaflets; and
wherein after a radial expansion of the valve stent (1), the valve clamping and securing apparatus (2) is in the second form, and the radial expansion of the valve stent (1) causes the hoop member (3) to drive the autologous valve leaflets to move upward and tightly hoop the valve stent (1).

2. The prosthetic valve replacement system according to claim 1, wherein a far end of the valve clamping mechanism (21) is detachably connected, and under the first form, the valve clamping and securing apparatus (2) allows a valve leaflet around the valve clamping mechanism (21) to be able to open and close autonomously, when the valve stent (1) enters the valve clamping mechanism (21), the far end of the valve clamping mechanism (21) is detached, and when the valve stent (1) is in a radial expansion state, the radial expansion of the valve stent (1) causes the hoop member (3) to drive the autologous valve leaflets to move upward and tightly hoop the valve stent (1).

3. The prosthetic valve replacement system according to claim 1, wherein the radial expansion of the valve stent (1) drives the valve clamping mechanism (21), the hoop member (3), the autologous valve leaflets and chordae tendineae in linkage, the hoop member (3) is at least partially moved upward and drives the autologous valve leaflets and the chordae tendineae to be lifted upward, and at least one of the autologous valve leaflets or chordae tendineae tissue is hooped between the hoop member (3) and the valve stent (1).

4. The prosthetic valve replacement system according to claim 1, wherein under the first form, the valve clamping mechanism (21) clamps a portion of each of the autologous valve leaflets, and under the second form, the autologous valve leaflets around the valve clamping mechanism (21) are driven by the hoop member (3) to move upward.

5. The prosthetic valve replacement system according to claim 1, further comprising an inner core tube (4), wherein a far end of the inner core tube (4) cooperates with and is detachably connected to a far end of the valve clamping mechanism (21), and the valve stent (1) enters the valve clamping mechanism (21) along the inner core tube (4).

6. The prosthetic valve replacement system according to claim 1, wherein the hoop member (3) and the valve clamping mechanism (21) comprise at least two connecting points, and the at least two connecting points of the hoop member (3) and the valve clamping mechanism (21) have different heights.

7. The prosthetic valve replacement system according to claim 1, wherein the valve clamping and securing apparatus (2) comprises a single valve clamp (10), an atrial support segment (101), a ventricular segment (102) and the hoop member (3), wherein the hoop member (3) is connected to the single valve clamp (10) and the ventricular segment (102), and the hoop member (3) has an inclined form on a cross section parallel to a central axis of the valve clamping and securing apparatus (2).

8. The prosthetic valve replacement system according to claim 1, wherein the valve clamping mechanism (21) comprises a plurality of clamps, and connecting points of the hoop member (3) and the plurality of clamps have different heights to enable the hoop member (3) to have a waved form under the second form.

9. The prosthetic valve replacement system according to claim 5, wherein the valve clamping mechanism (21) comprises a first clamping half part (211) and a second clamping half part (212), the first clamping half part (211) and the second clamping half part (212) are detachably connected or are two separate members separated from each other, and the first clamping half part (211) and the second clamping half part (212) are configured to capture and clamp different autologous valve leaflets respectively.

10. The prosthetic valve replacement system according to claim 9, wherein the first clamping half part (211) and the second clamping half part (212) have complementary shapes and are detachably buckled through the inner core tube (4) under a restraint state of the valve clamping and securing apparatus (2).

11. The prosthetic valve replacement system according to claim 1, wherein a positioning ring (5, 11) positioned within an atrium is further disposed on the valve stent (1), is configured to have a form that adapts to a physiological structure of an autologous valve annulus and is provided with an auxiliary securing rod.

12. The prosthetic valve replacement system according to claim 1, wherein a securing clamp (9) is disposed on the valve stent (1), and under the second form, the securing clamp (9) clamps the autologous valve leaflets and enables the autologous valve leaflets to be clamped between the valve stent (1) and the securing clamp (9).

## Patentansprüche

1. Prothesenklappenersatzsystem, umfassend einen Klappenstent (1) und eine mit dem Klappenstent (1) zusammenwirkende Klappenklemm- und -sicherungsvorrichtung (2), wobei die Klappenklemm- und -sicherungsvorrichtung (2) als Klappenklemmmechanismus (21) ausgebildet ist, und ein Umschließungselement (3) zum Klemmen autologer Klappensegel, und wobei das Umschließungselement (3) zumindest teilweise an dem Klappenklemmmechanismus (21) angeordnet ist,
wobei die Klappenklemm- und -sicherungsvorrichtung (2) ausgelegt ist, um fähig zu sein, eine erste Form und eine zweite Form aufzuweisen, und wobei die zweite Form nach der ersten Form vorhanden ist,
wobei der Klappenklemmmechanismus (21), wenn sich die Klappenklemm- und - sicherungsvorrichtung (2) in der ersten Form befindet, ausgelegt ist, um fähig zu sein, die autologen Klappensegel zu greifen und zu klemmen, und
wobei sich die Klappenklemm- und -sicherungsvorrichtung (2) nach einer radialen Expansion des Klappenstents (1) in der zweiten Form befindet und die radiale Expansion des Klappenstents (1) das Umschließungselement (3) veranlasst, die autologen Klappensegel nach oben in Bewegung zu versetzen und den Klappenstent (1) fest zu umschließen.

2. Prothesenklappenersatzsystem nach Anspruch 1, wobei ein entferntes Ende des Klappenklemmmechanismus (21) lösbar verbunden ist und die Klappenklemm- und - sicherungsvorrichtung (2) in der ersten Form einem Klappensegel rund um den Klappenmechanismus (21) ermöglicht, fähig zu sein, sich eigenständig zu öffnen und zu schließen, wenn der Klappenstent (1) in den Klappenklemmmechanismus (21) eintritt, wobei das entfernte Ende des Klappenklemmmechanismus (21) gelöst ist, und die radiale Expansion des Klappenstents (1), wenn sich der Klappenstent (1) in einem Zustand der radialen Expansion befindet, das Umschließungselement (3) veranlasst, die autologen Klappensegel nach oben in Bewegung zu versetzen und den Klappenstent (1) fest zu umschließen.

3. Prothesenklappenersatzsystem nach Anspruch 1, wobei die radiale Expansion des Klappenstents (1) eine Kopplung des Klappenklemmmechanismus (21), des Umschließungselements (3), der autologen Klappensegel und der Sehnenfäden bewirkt, wobei das Umschließungselement (3) zumindest teilweise nach oben bewegt wird und bewirkt, dass die autologen Klappensegel und die Sehnenfäden nach oben angehoben werden und mindestens entweder die autologen Klappensegel oder das Sehnenfädengewebe zwischen dem Umschließungselement (3) und dem Klappenstent (1) umschlossen werden.

4. Prothesenklappenersatzsystem nach Anspruch 1, wobei der Klappenklemmmechanismus (21) in der ersten Form einen Abschnitt eines jeden der autologen Klappensegel klemmt und die autologen Klappensegel rund um den Klappenklemmmechanismus (21) in der zweiten Form durch das Umschließungselement (3) nach oben in Bewegung versetzt werden.

5. Prothesenklappenersatzsystem nach Anspruch 1, ferner umfassend ein Innenkernrohr (4), wobei ein entferntes Ende des Innenkernrohrs (4) mit einem entfernten Ende des Klappenklemmmechanismus (21) zusammenwirkt und lösbar mit diesem verbunden ist und der Klappenstent (1) entlang des Innenkernrohrs (4) in den Klappenklemmmechanismus (21) eintritt.

6. Prothesenklappenersatzsystem nach Anspruch 1, wobei das Umschließungselement (3) und der Klappenklemmmechanismus (21) mindestens zwei Verbindungspunkte umfassen und die mindestens zwei Verbindungspunkte des Umschließungselements (3) und des Klappenklemmmechanismus (21) unterschiedliche Höhen aufweisen.

7. Prothesenklappenersatzsystem nach Anspruch 1, wobei die Klappenklemm- und -sicherungsvorrichtung (2) eine einzelne Klappenklemme (10), ein atriales Stützsegment (101), ein ventrikuläres Segment (102) und das Umschließungselement (3) umfasst, wobei das Umschließungselement (3) mit der einzelnen Klappenklemme (10) und dem ventrikulären Segment (102) verbunden ist und das Umschließungselement (3) eine geneigte Form auf einem Querschnitt parallel zu einer zentralen Achse der Klappenklemm- und -sicherungsvorrichtung (2) aufweist.

8. Prothesenklappenersatzsystem nach Anspruch 1, wobei der Klappenklemmmechanismus (21) eine Vielzahl von Klemmen umfasst und Verbindungspunkte des Umschließungselements (3) und der Vielzahl von Klemmen unterschiedliche Höhen aufweisen, um dem Umschließungselement (3) zu ermöglichen, in der zweiten Form eine gewellte Form aufzuweisen.

9. Prothesenklappenersatzsystem nach Anspruch 5, wobei der Klappenklemmmechanismus (21) eine erste Klemmhälfte (211) und eine zweite Klemmhälfte (212) umfasst, wobei die erste Klemmhälfte (211) und die zweite Klemmhälfte (212) lösbar verbunden oder zwei separate, voneinander getrennte Elemente sind und die erste Klemmhälfte (211) und die zweite Klemmhälfte (212) ausgelegt sind, um jeweils unterschiedliche autologe Klappensegel zu greifen und zu klemmen.

10. Prothesenklappenersatzsystem nach Anspruch 9, wobei die erste Klemmhälfte (211) und die zweite Klemmhälfte (212) einander ergänzend ausgestaltet sind und in einem zurückgehaltenen Zustand der Klappenklemm- und -sicherungsvorrichtung (2) durch das Innenkernrohr (4) lösbar verrastet sind.

11. Prothesenklappenersatzsystem nach Anspruch 1, wobei ein Positionierungsring (5, 11), der innerhalb eines Vorhofs positioniert ist, ferner auf dem Klappenstent (1) angeordnet und ausgelegt ist, um eine Form aufzuweisen, die sich einer physiologischen Struktur eines autologen Klappenanulus anpasst, und mit einem Hilfssicherungsstab versehen ist.

12. Prothesenklappenersatzsystem nach Anspruch 1, wobei eine Sicherungsklemme (9) auf dem Klappenstent (1) angeordnet ist und die Sicherungsklemme (9) in der zweiten Form die autologen Klappensegel klemmt und den autologen Klappensegeln ermöglicht, zwischen dem Klappenstent (1) und der Sicherungsklemme (9) geklemmt zu werden.

## Revendications

1. Système de remplacement de valve prothétique, comprenant un stent de valve (1) et un appareil de serrage et de fixation de valve (2) coopérant avec le stent de valve (1), dans lequel l'appareil de serrage et de fixation de valve (2) est construit sous la forme d'un mécanisme de serrage de valve (21) et d'un élément arceau (3) pour serrer des feuillets de valve autologues, et l'élément arceau (3) est disposé au moins partiellement sur le mécanisme de serrage de valve (21),
dans lequel l'appareil de serrage et de fixation de valve (2) est configuré pour pouvoir présenter une première forme et une deuxième forme, et la deuxième forme se manifeste après la première forme,
dans lequel lorsque l'appareil de serrage et de fixation de valve (2) est dans la première forme, le mécanisme de serrage de valve (21) est configuré pour pouvoir capturer et serrer les feuillets de valve autologues ; et
dans lequel, après une expansion radiale du stent de valve (1), l'appareil de serrage et de fixation de valve (2) est dans la deuxième forme, et l'expansion radiale du stent de valve (1) amène l'élément arceau (3) à entraîner les feuillets de valve autologues en mouvement vers le haut et à entourer étroitement le stent de valve (1).

2. Système de remplacement de valve prothétique selon la revendication 1, dans lequel une extrémité distante du mécanisme de serrage de valve (21) est reliée de manière amovible, et sous la première forme, l'appareil de serrage et de fixation de valve (2) permet à un feuillet de valve autour du mécanisme de serrage de valve (21) de s'ouvrir et de se fermer de façon autonome, lorsque le stent de valve (1) pénètre dans le mécanisme de serrage de valve (21), l'extrémité distante du mécanisme de serrage de valve (21) est détachée, et lorsque le stent de valve (1) est dans un état d'expansion radiale, l'expansion radiale du stent de valve (1) amène l'élément arceau (3) à entraîner les feuillets de valve autologues en mouvement vers le haut et à entourer étroitement le stent de valve (1).

3. Système de remplacement de valve prothétique selon la revendication 1, dans lequel l'expansion radiale du stent de valve (1) entraîne le mécanisme de serrage de valve (21), l'élément arceau (3), les feuillets de valve autologues et les cordages tendineux de manière solidaire, l'élément arceau (3) est au moins partiellement déplacé vers le haut et entraîne les feuillets de valve autologues et les cordages tendineux à se soulever vers le haut, et au moins l'un des feuillets de valve autologues ou du tissu de cordages tendineux est encerclé entre l'élément arceau (3) et le stent de valve (1).

4. Système de remplacement de valve prothétique selon la revendication 1, dans lequel sous la première forme, le mécanisme de serrage de valve (21) serre une partie de chacun des feuillets de valve autologues, et sous la deuxième forme, les feuillets de valve autologues autour du mécanisme de serrage de valve (21) sont entraînés par l'élément arceau (3) en mouvement vers le haut.

5. Système de remplacement de valve prothétique selon la revendication 1, comprenant en outre un tube âme interne (4), dans lequel une extrémité distante du tube âme interne (4) coopère avec et est reliée de manière amovible à une extrémité distante du mécanisme de serrage de valve (21), et le stent de valve (1) pénètre dans le mécanisme de serrage de valve (21) le long du tube âme interne (4).

6. Système de remplacement de valve prothétique selon la revendication 1, dans lequel l'élément arceau (3) et le mécanisme de serrage de valve (21) comprennent au moins deux points de connexion, et les au moins deux points de connexion de l'élément arceau (3) et du mécanisme de serrage de valve (21) présentent des hauteurs différentes.

7. Système de remplacement de valve prothétique selon la revendication 1, dans lequel l'appareil de serrage et de fixation de valve (2) comprend une pince de valve unique (10), un segment de support auriculaire (101), un segment ventriculaire (102) et l'élément arceau (3), dans lequel l'élément arceau (3) est relié à la pince de valve unique (10) et au segment ventriculaire (102), et l'élément arceau (3) présente une forme inclinée sur une section transversale parallèle à un axe central de l'appareil de serrage et de fixation de valve (2).

8. Système de remplacement de valve prothétique selon la revendication 1, dans lequel le mécanisme de serrage de valve (21) comprend une pluralité de pinces, et des points de connexion de l'élément arceau (3) et de la pluralité de pinces présentent des hauteurs différentes pour permettre à l'élément arceau (3) de présenter une forme ondulée sous la deuxième forme.

9. Système de remplacement de valve prothétique selon la revendication 5, dans lequel le mécanisme de serrage de valve (21) comprend une première demi-partie de serrage (211) et une deuxième demi-partie de serrage (212), la première demi-partie de serrage (211) et la deuxième demi-partie de serrage (212) sont reliées de manière amovible ou sont deux éléments distincts séparés l'un de l'autre, et la première demi-partie de serrage (211) et la deuxième demi-partie de serrage (212) sont configurées pour capturer et serrer des feuillets de valve autologues différents respectivement.

10. Système de remplacement de valve prothétique selon la revendication 9, dans lequel la première demi-partie de serrage (211) et la deuxième demi-partie de serrage (212) présentent des formes complémentaires et sont verrouillées de manière amovible à travers le tube âme interne (4) dans un état de retenue de l'appareil de serrage et de fixation de valve (2).

11. Système de remplacement de valve prothétique selon la revendication 1, dans lequel un anneau de positionnement (5, 11), positionné à l'intérieur d'un atrium est en outre disposé sur le stent de valve (1) et est configuré pour présenter une forme qui s'adapte à une structure physiologique d'un anneau de valve autologue et est pourvu d'une tige de fixation auxiliaire.

12. Système de remplacement de valve prothétique selon la revendication 1, dans lequel une pince de fixation (9) est disposée sur le stent de valve (1), et sous la deuxième forme, la pince de fixation (9) serre les feuillets de valve autologues et permet aux feuillets de valve autologues d'être serrés entre le stent de valve (1) et la pince de fixation (9).
